(19) Europäisches Patentamt
European Patent Office
Office européen des brevets

(11) Publication number: **0 393 893 B1**

# (12) EUROPEAN PATENT SPECIFICATION

(45) Date of publication of patent specification: **11.01.95**

(51) Int. Cl.[6]: **C07C 381/12**, C08F 2/50

(21) Application number: **90303748.9**

(22) Date of filing: **09.04.90**

## (54) Sulfonium compound and polymerisation initator comprising the sulfonium compound as the main ingredient.

(30) Priority: **08.04.89 JP 89517/89**
**12.04.89 JP 93660/89**
**13.10.89 JP 267420/89**
**13.10.89 JP 267421/89**

(43) Date of publication of application:
**24.10.90 Bulletin 90/43**

(45) Publication of the grant of the patent:
**11.01.95 Bulletin 95/02**

(84) Designated Contracting States:
**CH DE FR GB IT LI NL**

(56) References cited:
**EP-A- 0 331 496**
**US-A- 4 034 046**

(73) Proprietor: **SANSHIN KAGAKU KOGYO CO., LTD.**
**150, Oaza Yanai**
**Yanai-shi**
**Yamaguchi-ken (JP)**

(72) Inventor: **Hamazu, Fumio**
**911-4, Oaza Ogo,**
**Tabuse-cho**
**Kumage-gun,**
**Yamaguchi-ken (JP)**
Inventor: **Koizumi, Tatsuya**
**742-1, Oaza Hiraoson,**
**Hiorao-cho**
**Kumage-gun,**
**Yamaguchi-ken (JP)**
Inventor: **Endo, Takeshi**
**54-13 Miyagatani,**
**Nishi-ku**
**Yokohama-shi,**
**Kanagawa-ken (JP)**
Inventor: **Yamamoto, Yoshinari**
**75, Ogodanchi,**
**Tabuse-cho**
**Kumage-gun,**
**Yamaguchi-ken (JP)**

(74) Representative: **Denmark, James**
**Bailey, Walsh & Co.**
**5 York Place**
**Leeds LS1 2SD**
**Yorkshire (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention concerns a novel sulfonium compound, a polymerization initiator comprising the sulfonium compound as the main ingredient and a polymerization process using the polymerization initiator More specifically, the present invention relates to a cationically polymerizable composition containing a novel sulfonium compound useful for obtaining a curing product having satisfactory property or an oligomer of a cationically polymerizable vinyl compound having practical usefulness by polymerizing cationically polymerizable material in a short period of time by means of heat or radiation rays such as light or electron beam, a curing agent for such polymerization and a polymerization process.

Generally, cationically polymerizable materials, among all, epoxy resins have been employed for various application uses requiring materials of high performance. Particularly, since the method of curing by polymerization under the irradiation of radiation rays at predetermined wavelength such as ultraviolet rays can provide curing within a short period of time by a simple polymerization process, it has been used for various application uses.

Epoxy resins have so far been used generally in the form of a two-component system by incorporating an active amine-containing compound or a carboxylic acid anhydride as a curing agent. However, the two-component system using such a curing agent involves a drawback that the ingredients have to be mixed completely and it needs curing time as long as several hours.

In addition, there is a boron trifluoride-monoethyl amine system for curing the epoxy resin in a one component system but it takes from 1 to 8 hours even at a high temperature of 160 °C or higher. As an improvement, although there can be mentioned U.S. Patent No. 3565861, it also requires high temperature. Accordingly, these compositions can not be used for the coating of heat sensitive electronic parts, etc. Further, in a case of curing an epoxy resin by light, there has been proposed a method of using an aryl diazonium metal halide complex. However, since polymerization is taken place rapidly and premature gelation tends to occur in this method, it may be considered that the composition can not be stored satisfactorily even for a short period of time if it is in a dark place. In view of the above, it has been proposed to suppress the premature gelation of the composition and provide storage-stability in a dark place for epoxy material containing metal halide salt of an aryl diazonium salt. However, since nitrogen is released during curing from such a composition, the resultant curing material involves a problem of incorporating bubbles, as well as the cost is increased and completely satisfactory results have not yet been obtained.

By the way, various photodegrading sulfonium salts have been known for the onium salt. For instance, it has been known to use p-hydroxyphenyl dimethylsulfonium hexafluoro arsenates as a photocuring agent in Japanese Patent Laid-Open No. Sho 54-53181 and phenyl sulfonium salt as a photocuring agent for epoxy resins in Japanese Patent Laid-Open No. Sho 50-151997. Further, there has been disclosure in Research Disclosure May 1988, p 298 - 299 of using p-cyanobenzylmethyl-9-anthryl sulfonium trifluoromethanesulfonate, etc. as a photocuring agent. However, although such onium salts can photocure epoxy resins, epoxy resins can not be cured by merely applying heat. Further, a photopolymerization initiator that changes its dissolving behavior by radiation rays has been disclosed in Japanese Patent Laid-Open No. Sho 64-26550.

On the other hand, as the thermal latent type initiator capable of activating onium salts by applying heat thereby curing epoxy resins, there have been reported benzyl sulfonium salt, for example, dialkylbenzyl sulfonium hexafluoroantimonate in Japanese Patent Laid-Open Nos. Sho 58-37003, 63-223002 and a trialkyl sulfonium salt in Japanese Patent Laid-Open No. Sho 56-162833, and a dialkyl hydroxyaryl sulfonium salt in Japanese Patent Publication Nos. 56-500218 and 56-500496. However, these sulfonium salts are inert to light and can not cure epoxy resins in a short period of time, as well as require temperature, for example, 150 °C or higher, and can not be said practical both in view of the heating time and the temperature.

Further, Japanese Patent Laid-Open No. 60-188425 discloses a curing agent for the heat polymerization of special esters such as spiro type esters.

On the other hand, as the cationic polymerization catalyst for cationically polymerizable vinyl compounds, there have been known Lewis acid catalyst such as mineral acid, $BF_3$, $ZnCl_2$, $AlCl_3$ and halogen-containing organic aluminum compound such as $AlR_2Cl$ and $AlRCl_2$. However, since these catalysts cause too violent cationic polymerization reaction at a temperature of 0 °C or higher polymerization reaction can not be controlled as desired and polymerization degree can not be increased. It is of course impossible to previously formulate a cationically polymerizable monomer and such a catalyst into one-part system at a room temperature for storage.

For polymerizing a vinyl compound, it is necessary for controlling the chain transfer reaction or termination reaction and, accordingly, polymerization reaction has usually been conducted by charging a catalyst solution after cooling a cationically polymerizable monomer dissolved in an appropriate solvent to a

predetermined extremely low temperature. However, for conducting the polymerizing reaction industrially at an extremely low temperature (for instance, from -130 °C to -40 °C), since it is necessary to use a cooling medium at low temperature, this makes the operation complicate and increases the cooling cost and, accordingly, it can not be said to be quite satisfactory. Although US. Patent No. 3283010 describes the release of benzyl cations, polymerization for the polymer is not intended in this patent.

Further, in view of compounds, p-hydroxyphenyl benzylsulfonium compound is disclosed in Japanese Patent Laid-Open Sho 50-29511 and p-hydroxyphenyl benzylsulfonium halide is disclosed in U.S. Patent No. 4034046. However, substituted or not-substituted oxyphenyl benzyl or naphthylmethyl alkyl sulfonium (sub)metal polyfluoride complex has not yet been known.

OBJECT AND SUMMARY OF THE INVENTION

The object of the present invention is to provide a cationically polymerizable composition containing a novel sulfonium compound capable of polymerizing cationically polymerizable materials such as epoxy resins in a short period of time under the irradiation of heat or radiation rays such as light or electron rays, and utilizable as sealant, matrix resin for composite material, etc., a polymerization initiator comprising the novel sulfonium compound as the main ingredient, as well as a polymerization process using such a polymerization initiator.

Another object of the present invention is to provide a monomer composition capable of polymerizing a cationically polymerizable vinyl compound such as styrene monomer under a certaincondition and excellent in storage stability as a mixture with polymerization initiator, industrially provide an oligomer of a vinyl compound of practical usefulness, as well as provide a polymerization process therefor and a catalyst as a novel compound.

The novel compound referred to in the present invention is a sulfonium compound represented by the general formulae (I) or (II) :

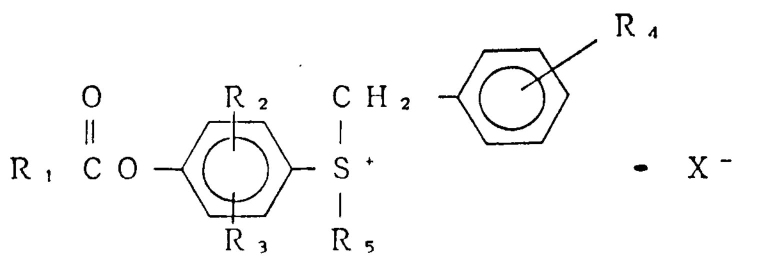
...... ( I )

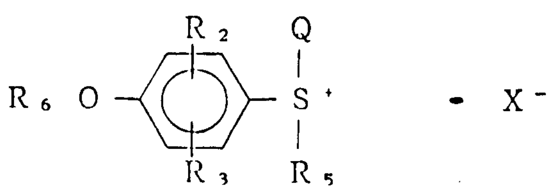
......... ( II )

where $R_1$ represents ethoxy group, phenyl group, benzyloxy group, chloromethyl group, phenoxy group, 9-fluorenylmethoxy group or trichloromethyl group, $R_2$ and $R_3$ represent, independently, hydrogen, halogen or $C_1$ - $C_4$ alkyl group, $R_4$ represents hydrogen or methoxy group, $R_5$ represents $C_1$ - $C_4$ alkyl group, $R_6$ represents hydrogen, methyl group, acetyl group, methoxy carbonyl group, ethoxy carbonyl group, chloroacetyl group, trichloroacetyl group, benzyloxy carbonyl group, benzoyl group, phenoxy carbonyl group or 9-fluorenyl methoxy carbonyl group, Q represents chlorobenzyl group, methyl benzyl group, nitrobenzyl group, dinitrobenzyl group, $\alpha$-naphthylmethyl group or $\beta$-naphthylmethyl group and X represents $SbF_6$, $PF_6$, $AsF_6$ or $BF_4$, providing that if $R_6$ is hydrogen, methyl group, acetyl group or methoxycarbonyl group, Q does not include chlorobenzyl group or methylbenzyl group.

As has been described above, the present invention comprises essentially substituted or not-substituted oxyphenyl, substituted or not-substituted benzyl or naphthylmethyl alkyl sulfonium (sub)metal polyfluoride complex in which the novelty of the invention is present. The present compound is synthesized by using a corresponding sulfonium chloride or sulfonium methylsulfate as the starting material and reacting it with an

alkali metal salt of a predetermined acid, for example, $NaSbF_6$, $KSbF_6$, $NaBF_4$, $LiBF_4$, $NaPF_6$, $KPF_6$, $NaAsF_6$, $KAsF_6$ in a predetermined anhydrous or hydrous organic solvent. The organic solvent used in this case is selected from methanol, acetone, ethyl acetate, ethanol and acetonitrile. Other solvents than described above, for example, benzene or toluene cause no reaction since they do not substantially dissolve the inorganic salt. Further, although DMF or DMSO can proceed the reaction due to their solubility, they are difficult to be removed from the reaction system owing to their high boiling point.

As the second synthesis method, it is also proposed a method of obtaining a substituted oxyphenyl benzyl/naphthylmethyl sulfonium compound by reacting the hydroxy group of a hydroxyphenyl benzyl/naphthylmethyl alkyl sulfonium compound with an acid halide such as chloromethyl carbonate, acetyl chloride or acetyl iodine under the presence of tertiary amine. The reaction solvent in this method includes ethyl acetate and acetonitrile. Other solvents can not provide preferred result. For instance, protonic solvents such as water, methanol or ethanol react with acid halide. Aromatic solvents typically represented by benzene do not dissolve the product and, accordingly, reduce the purity thereof. Polar solvents typically represented by the DMF or DMSO can provide reaction, but it is difficult to remove them from the reaction system due to their high boiling point, causing, for example, decomposition of products during removal. The reaction temperature is preferably 20 °C or lower, particularly preferably, 5 °C or lower for avoiding the decomposition of the product. The tertiary amine added as a dehydrogen halide agent is preferably triethylamine, trimethylamine, N-methylmorpholine, etc., which may be used alone or as a mixture of two or more of them.

The polymerization initiator according to the present invention comprises a paired compound of a (substituted or not-substituted)-benzyl/naphthylmethyl-4-(substituted or not-substituted)-oxyphenyl solfonium cation and a predetermined anion represented by the general formula (I) or (II) as the constituent factor.

The catinically polymerizable material used in the present invention is an acid polymerizable or acid curable material and epoxy resins are used particularly preferably. Examples of suitable material are epoxide monomers, episulfide monomers, polyepoxides (or epoxy resin), polyepisulfides (or episulfide resins), phenyl/formaldehyde resin, melamine/formaldehyde resin, urea/formaldehyde resin, cyclic ether and thiocyclic ether such as spiro-orthoester, bicycloorthoester and spiro-ortho carbonate (other than epoxide and episulfide) and polymers thereof, lactone, styrene, vinyl ether and vinyl thioether, as well as those resins containing a crosslinker for crosslinking or curing resins when treated with acid, which may be used alone or as a mixture of two or more of them.

Preferred benzyl sulfonium salt used as the polymerization catalyst in the present invention can include, for example,

o-nitrobenzyl-4-hydroxphenyolmethylsulfonium hexafluoroantimonate,
m-nitrobenzyl-4-hydroxyphenylmethylsulfonium hexafluoroantimonate,
o-nitrobenzyl-4-hydroxyphenylmethylsulfonium hexafluorophosphate,
m-nitrobenzyl-4-hydroxyphenylmethylsulfonium hexafluoroarsenate,
3,5-dinitrobenzyl-4-hydroxyphenylmethylsulfonium hexafluoroantimonate,
3,5-dinitrobenzyl-4-hydroxyphenylethylsulfonium hexafluoroantimonate,
o-nitrobenzyl-4-hydroxyphenylethylsulfonium hexafluoroantimonate,
o-nitrobenzyl-3-chlor-4-hydroxyphenylmethylsulfonium hexafluoroantimonate,
2,4,6-trinitrobenzyl-4-hydroxyphenylmethylsulfonium hexafluoroantimonate,
2,4-dinitrobenzyl-4-hydroxyphenylmethylsulfonium hexafluorophosphate,
$\alpha$-naphthylmethyl-4-hydroxyphenylmethylsulfonium hexafluoroantimonate,
$\beta$-naphthylmethyl-4-hydroxyphenylmethylsulfonium hexafluoroantimonate,
$\alpha$-naphthylmethyl-4-hydroxyphenylmethylsulfonium hexafluorophosphate,
$\alpha$-naphthylmethyl-4-(benzyloxycarbonyloxy)phenylmethylsulfonium hexafluoroantimonate,
$\alpha$-naphthylmethyl-4-(methoxycarbonyloxy)phenylmethylsulfonium hexafluoroantimonate,
4-(benzoyloxy)phenylbenzylmethylsulfonium hexafluoroantimonate
4-(benzoyloxy)phenylbenzylethylsulfonium hexafluoroantimonate,
4-(benzoyloxy)phenylbenzylmethylsulfonium hexafluorophosphate,
4-(benzoyloxy)phenylbenzylmethylsulfonium tetrafluoroborate,
4-(benzoyloxy)phenylbenzylmethylsulfonium hexafluoroarsenate,
benzyl-4-(ethoxycarbonyloxy)phenylmethylsulfonium hexafluoroantimonate,
benzyl-4-(ethoxycarbonyloxy)phenylmethylsulfonium hexafluorophosphate,
benzyl-4-(benzyloxycarbonyloxy)phenylmethylsulfonium hexafluoroantimonate,
benzyl-4-(phenoxycarbonyoxy)phenylmethylsulfonium hexafluoroantimonate,
benzyl-3-chloro-4-(benzoyloxy)phenylmethylsulfonium hexafluorophosphate,
p-methylbenzyl-4-(benzyloxycarbonyloxy)phenylmethylsulfonium hexafluoroantimonate,

p-methylbenzyl-4-chloracetoxyphenylmethylsulfonium hexafluroantimonate,
p-nitrobenzyl-4-hydroxyphenylmethylsulfonium hexafluoroantimonate, and
p-nitrobenzyl-3-methyl-4-hydroxyphenylethylsulfonium hexafluoroantimonate,

The sulfonium salts used in the present invention increase the activity to heat, as well as have an activity to radiation rays such as light or electron beam. That is, it is considered that such benzyl sulfonium salts excited by heat or radiation rays release benzyl cations or naphthylmethyl cations thereby proceeding the polymerization of the cationicaly polymerizable material described above.

Further, in the present invention, in a case where the cationically polymerizable material is a vinyl compound, solution polymerization is usually conducted by using a solvent inert to the catalyst or the monomer upon polymerization reaction, but bulk polymerization is also employed. As the solution usable herein, there can be mentioned, for example, aromatic hydrocarbons such as benzene, toluene and xylene, aliphatic hydrocarbons such as n-hexane and n-heptane, cycloaliphatic hydrocarbons such as cyclohexane, hydrocarbon mixtures such as petroleum ether and ligroin, and halogenated hydrocarbons such as chlorobenzene and dichloroethane. The polymerizing reaction for the vinyl compounds is conducted under a normal or elevated pressure at a reaction temperature of 20 °C or higher, preferably, from 30 to 150 °C at which heating can easily be conducted industrially.

Further, since the sulfonium salt used as the polymerization initiator in the present invention does not start the cationic polymerization reaction, for instance p-methylbenzyl-4-substituted oxy or 4-hydroxyphenylmethylsulfonium salt in a cold place and α-naphthylmethyl-4-substituted oxy or 4-hydroxyphenylmethylsulfonium salt in a dark and cold place, it is also possible, if required, to form the monomer and the initiator previously into a one component system by using an appropriate solvent and store them.

The sulfonium salt used in the present invention is used from 0.01 to 20 parts by weight, preferably, from 0.1 to 10 parts by weight based on 100 parts by weight of a resin. If it is less than 0.01 parts by weight, no sufficient polymer can be obtained. With an addition amount in excess of 20 parts by weight, no preferred products can be obtained in view of the physical property after the polymerization and it is neither preferred in view of the cost. Further, polymerizatin reaction or curing reaction is conducted by treatment with radiation rays or heat and, if required, heating and irradiation of radiation rays may be used in combination. Further, a solvent may be used, if required, upon polymerization.

As the radiation rays in the present invention, ultraviolet rays at a wavelength within a range from 200 to 400 nm show best efficiency and, accordingly, there can be mentioned low pressure mercury lamp, medium pressure mercury lamp, high pressure mercury lamp, xenone lamp, carbon arc lamp, etc. as the light source and, further, polymerization reaction or curing reaction may be conducted by the irradiation of electron beam or sunlight.

The composition according to the present invention may be used, depending on the case, in admixture with auxiliary agents typically represented by extender, flame retardant, antistatic agent, surface active agent and acid anhydride, and it can be used for lustering varnish, ink, paint, adhesive, laminated board,prepreg, molding material, sealing material, etc.

Since the curable composition according to the present invention does not start the cationic polymerization, for example, p-methylbenzyl-4-substituted oxy or 4-hydroxyphenylmethylsulfonium salt in a cold place and α-naphthylmethyl-4-substituted oxy or 4-hydroxyphenylmethylsulfonium salt in a dark and cold place, it can be stored for a long period of time and has a function of rapidly starting the polymerization under the irradiation of radiation rays such as light or electron beam, heating at a temperature of 150 °C or lower or by the combined use of heating and radiation treatment, and can provide curing products of excellent curability at high temperature, with no hydroscopic property and excellent in water proofness, chemical resistance and electrical property.

Further, the cationically polymerizable vinyl compound composition has a feature capable of storing the monomer and the catalyst in one-part system which has been difficult so far, enabling cation polymerization at a temperature of 20 °C or higher and obtaining a polymer at high polymerization degree of practical usefulness by simple heating.

## DETAILED DESCRIPTION OF THE PREFERRED EMBODIMENTS

The present invention is to be described referring to preferred embodiment but it should be noted that the invention is not limited only to the following examples.

Synthetic Example 1

Synthesis for o-nitrobenzyl-4-hydroxyphenylmethylsulfonium hexafluoroantimonate

o-Nitrobenzyl-4-hydroxyphenylmethylsulfonium chloride was dissolved in an amount of 7.50 g (0.024 mol) into 500 ml of methanol, to which 6.60 g (0.024 mol) of powder of $KSbF_6$ was added under stirring and they were stirred further for one hour. The solution was concentrated under a reduced pressure and the residue was extracted. The extract was washed with water, dried and then concentrated to obtain 9.50 g (77.2% yield) of the aimed product as white crystals from the residue.

Melting point 121.0~124.0 °C
IR (KBr) $cm^{-1}$
3480, 1530, 1345, 675
NMR (Acetone-$d_6$) ppm
$\delta$ = 3.63 (3H, S, $C\underline{H}_3S^+<$)
$\delta$ = 5.35 (2H, dd, -$C\underline{H}_2C_6H_4$-)
$\delta$ = 7.01~8.30 (8H, m, -$C_6\underline{H}_4$- × 2)
$\delta$ = 9.80 (1H, S, $\underline{H}$O-)

| Elementary analysis $C_{14}H_{14}NO_3SSbF_6$ | | |
|---|---|---|
| Theoretical value<br>Measured value | C ; 32.84%,<br>C ; 32.60%, | H ; 2.76%<br>H ; 2.88% |

Synthetic Example 2

Synthetic for 3,5-dinitrobenzyl-4-hydroxyphenylmethylsulfonium hexafluoroantimonate

3,5-dinitrobenzyl-4-hydroxyphenylmethylsulfonium chloride was dissolved in an amount of 10 g (0.028 mol) into 900 ml of methanol, to which a powder of 7.7 g (0.028 mol) of $KSbF_6$ was added under stirring. Subsequently, the same procedures as those in Synthetic Example 1 were applied to obtain 11.0 g (70.5% yield) of the aimed product as yellow crystals.

Melting point 180.0~183.0 °C
IR (KBr) $cm^{-1}$
3475, 1545, 1350, 665
NMR (Acetone-$d_6$) ppm
$\delta$ = 3.57 (3H, S, $C\underline{H}_3S^+<$)
$\delta$ = 5.16-5.63 (2H, dd, -$C\underline{H}_2C_6H_3$-)
$\delta$ = 7.07-8.04 (4H, dd, $HOC_6\underline{H}_4$-)
$\delta$ = 8.57-8.62 (2H, d,

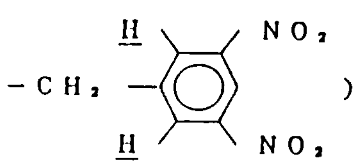

$\delta$ = 8.89-8.96 (1H, t,

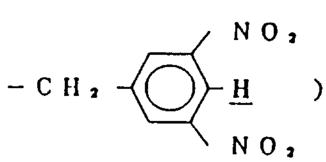

$\delta$ = 9.86 (1H, S, $\underline{H}$O-)

| Elementary analysis $C_{14}H_{13}O_5N_2SSbF_6$ | | |
|---|---|---|
| Theoretical value<br>Measured value | C ; 30.19%,<br>C ; 29.95%, | H ; 2.35%<br>H ; 2.31% |

Synthetic Example 3

Synthesis for α-naphthylmethyl-4-hydroxyphenylmethylsulfonium hexafluoroantimonate

α-naphthylmethyl-4-hydroxyphenylmethylsulfonium chloride was dissolved in an amount of 5.00 g (0.015 mol) into 200 ml of methanol, to which 4.08 g (0.015 mol) of powder of $KSbF_6$ was added under stirring. Subsequently, the same procedures as those in Synthesis Example 1 were applied o obtain 6.32 g (82.3% yield) of the aimed product as white crystals.

Melting point 125.5 °C (decomposition)
IR (KBr) $cm^{-1}$
3480, 665
NMR (Acetone-$d_6$) ppm
$\delta$ = 3.57 (3H, S, $\underline{CH_3}S^+$<)
$\delta$ = 5.23-5.91 (2H, dd, -$\underline{CH_2}C_{10}H_7$)
$\delta$ = 6.93~8.35 (11H, m, -$C_6\underline{H_4}$-, -$CH_2C_{10}\underline{H_7}$)
$\delta$ = 9.72 (1H, S, $\underline{H}$O-)

| Elementary analysis $C_{18}H_{17}OSSbF_6$ | | |
|---|---|---|
| Theoretical value<br>Measured value | C ; 41.81%,<br>C ; 41.63%, | H ; 3.31%<br>H ; 3.25% |

Synthetic Example 4

Synthesis for p-nitrobenzyl-4-hydroxyphenylmethylsulfonium hexafluoroantimonate

p-nitrobenzyl-4-hydroxyphenylmethylsulfonium hexafluoroantimonate was dissolved in an amount of 3.12 g (0.01 mol) into 300 ml of methanol, to which a powder of 2.75 g (0.01 mol) of $KSbF_6$ was added under stirring. Subsequently, the same procedures as those in Synthetic Example 1 were applied obtained 4.04 g (78.9% yield) of p-nitrobenzyl-4-hydroxyphenylmethylsulfonium hexafluoroantimonate as white crystals.

Melting point 165.0~167.0 °C
IR (KBr) $cm^{-1}$
3510, 665
NMR (Acetone-$d_6$) ppm
$\delta$ = 3.56 (3H, S, $\underline{CH_3}S^+$<)
$\delta$ = 4.99~5.49 (2H, dd, -$C_6H_4\underline{CH_2}$-)
$\delta$ = 7.01~8.30 (8H, m, -$C_6\underline{H_4}$- × 2)
$\delta$ = 9.79 (1H, S, $\underline{H}$O-)

| Elementary analysis $C_{14}H_{14}O_3NSSbF_6$ | | |
|---|---|---|
| Theoretical value<br>Measured value | C ; 32.84%,<br>C ; 32.73%, | H ; 2.76%<br>H ; 2.69% |

Synthetic Example 5

Synthesis for p-nitrobenzyl-4-hydroxyphenylmethylsulfonium hexafluorophosphate

p-nitrobenzyl-4-hydroxyphenylmethylsulfonium chloride was dissolved in an amount of 3.12 g (0.01 mol) into 300 ml of methanol, to which a powder of 1.84 g (0.01 mol) of $KPF_6$ was added under stirring. Subsequently, the same procedures as those in Synthetic Example 1 were applied to obtain 3.48 g (82.5% yield) of p-nitrobenzyl-4-hydroxyphenylmethylsulfonium hexafluorophosphate as white crystals.

Melting point 161.0~163.0 °C

IR (KBr) $cm^{-1}$

3500, 850

NMR (Acetone-$d_6$) ppm

$\delta$ = 3.50 (3H, S, $C\underline{H_3}S^+$<)

$\delta$ = 4.92~5.40 (2H, dd, -$C_6H_4C\underline{H_2}$-)

$\delta$ = 6.95~8.23 (8H, m, -$C_6\underline{H_4}$- × 2)

| Elementary analysis $C_{14}H_{14}O_3NSPF_6$ | | |
|---|---|---|
| Theoretical value | C ; 39.91%, | H ; 3.35% |
| Measured value | C ; 40.03%, | H ; 3.29% |

Synthetic Example 6

When reaction was conducted by using p-nitrobenzyl-4-hydroxyphenylmethylsulfonium bromide instead of p-nitrobenzyl-4-hydroxyphenylmethylsulfonium chloride in Synthetic Example 4, p-nitrobenzyl-4-hydroxyphenylmethylsulfonium hexafluoroantimonate was obtained at 65.2% yield.

Synthetic Example 7

Synthesis for benzyl-4-(ethoxycarbonyloxy)phenylmethylsulfonium hexafluoroantimonate

Benzyl-4-hydroxyphenylmethylsulfonium hexafluoroantimonate was dissolved in an amount of 20.0 g (0.043 mol) into 230 ml of ethyl acetate, to which 4.6 g (0.045 mol) of triethylamine was added at a temperature lower than 5 °C and 4.9 g (0.045 mol) of ethyl chloroformate was dropped at that temperature. After stirring for 3 hours, by produced triethylamine hydrogen chloride was removed by filtration and the acetonitrile layer was concentrated under a reduced pressure. The residue was recrystallzied to obtain 16.0 g (69.3% yield) of the aimed product as white crystals.

Melting Point 108.0~110.0 °C

IR (KBr) $cm^{-1}$

1770, 660

| Elementary analysis $C_{17}H_{19}O_3SSbF_6$ | | |
|---|---|---|
| Theoretical value | C ; 37.92%, | H ; 3.53% |
| Measured value | C ; 37.86%, | H ; 3.53% |

NMR (Acetone-$d_6$) ppm

$\delta$ = 1.35 (3H, t, J = 7Hz, $C\underline{H_3}CH_2$-)

$\delta$ = 3.50 (3H, S, $C\underline{H_3}S^+$<)

$\delta$ = 4.30 (2H, dd, J = 14Hz, 7Hz, $CH_3C\underline{H_2}$-)

$\delta$ = 5.12 (2H, dd, J = 16Hz, 12Hz, $C_6H_5C\underline{H_2}$-)

$\delta$ = 7.50 (2H, d, J = 9Hz)

= 7.94 (2H, d, J = 9Hz)

overall (4H, -$C_6\underline{H_4}$-)

$\delta$ = 7.33 (5H, S, $C_6\underline{H_5}$-)

Synthetic Example 8

Synthesis for benzyl-4-(phenoxycarbonyloxy)phenylmethylsulfonium hexafluoroantimonate

Benzyl-4-hydroxyphenylmethylsulfonium hexafluoroantimonate was dissolved in an amount of 20.0 g (0.043 mol) into 230 ml of acetonitrile, to which 4.6 g (0.045 mol) of triethylamine was added at a temperature lower than 5 °C and 7.1 g (0.045 mol) of phenyl chloroformate was dropped at that temperature. Subsequently, the same procedures as those in Synthetic Example 7 were applied to obtain 17.8 g (70.8% yield) of the aimed product as white crystals.

melting point 94.0~96.0 °C
IR (KBr) $cm^{-1}$
1770, 660

| Elementary analysis $C_{21}H_{19}O_3SSbF_6$ | | |
|---|---|---|
| Theoretical value | C ; 42.95%, | H ; 3.26% |
| Measured value | C ; 43.22%, | H ; 3.28% |

NMR (Acetone-$d_6$) ppm
$\delta$ = 3.50 (3H, S, $CH_3S^+$<)
$\delta$ = 5.12 (2H, dd, J = 16Hz, 12Hz, $C_6H_5CH_2$-)
$\delta$ = 7.35 (10H, S, $C_6H_5$- × 2)
$\delta$ = 7.63 (2H, d, J = 9Hz)
= 8.02 (2H, d, J = 9Hz)
overall (4H, -$C_6H_4$-)

Synthetic Example 9

Synthesis for benzyl-4-(benzoyloxy)phenylmethylsulfonium hexafluoroantimonate

Benzyl-4-hydroxyphenylmethylsulfonium hexafluoroantimonate was dissolved in an amount of 20.0 g (0.043 mol) into 230 ml of acetonitrile, to which 4.6 g (0.045 mol) of triethylamine was added at a temperature lower than 5 °C and 6.4 g (0.045 mol) of benzoyl chloride was dropped at that temperature. Subsequently, the same procedures as those in Synthetic Example 7 were applied to obtain 19.9 g (81.3% yield) of the aimed product as white crystals.

melting point 113.0~115.0 °C
IR (KBr) $cm^{-1}$
1750, 660

| Elementary analysis $C_{21}H_{19}O_2SSbF_6$ | | |
|---|---|---|
| Theoretical value | C ; 44.16%, | H ; 3.35% |
| Measured value | C ; 44.31%, | H ; 3.50% |

NMR (Acetone-$d_6$) ppm
$\delta$ = 3.53 (3H, S, $CH_3S^+$<)
$\delta$ = 5.17 (2H, dd, J = 16Hz, 12Hz, $C_6H_5CH_2$-)
$\delta$ = 7.36~8.23 (14H, m, $C_6H_5$- × 2, -$C_6H_4$-)

Synthetic Example 10

Synthesis for benzyl-4-(benzoyloxy)phenylmethylsulfonium hexafluorophosphate

Benzyl-4-hydroxyphenylmethylsulfonium hexafluorophosphate was dissolved in an amount of 20.0 g (0.053 mol) into 230 ml of acetonitrile, to which 5.7 g (0.056 mol) of triethylamine was added at a temperature lower than 5 °C and 7.9 g (0.056 mol) of benzoyl chloride was dropped at that temperature. Subsequently, the same procedures as those in Synthetic Example 7 were applied to obtain 20.6 g (80.6%

yield) of the aimed product as white crystals.

Melting point 146.0~146.5 °C

IR (KBr) $cm^{-1}$

1740 , 830

| Elementary analysis $C_{21}H_{19}O_2SPF_6$ | | |
|---|---|---|
| Theoretical value<br>Measured value | C ; 52.50%,<br>C ; 52.68%, | H ; 3.98%<br>H ; 3.96% |

NMR (Acetone-$d_6$) ppm

$\delta$ = 3.55 (3H, S, $\underline{CH_3}S^+$<)

$\delta$ = 5.16 (2H, dd, J = 16Hz, 12Hz, $C_6H_5\underline{CH_2}$-)

$\delta$ = 7.37~8.24 (14H, m, $C_6\underline{H_5}$- × 2, -$C_6\underline{H_4}$-)

Synthetic Example 11

Synthesis for benzyl-4-(benzyloxycarbonyloxy)phenylmethylsulfonium hexafluoroantimonate

Benzyl-4-hydroxyphenylmethylsulfonium hexafluoroantimonate was dissolved in an amount of 20.0 g (0.043 mol) into 230 ml of acetonitrile, to which 4.6 g (0.045 mol) of triethylamine was added at a temperature lower than 5 °C and 7.7 g (0.045 mol) of benzyl chloroformate was dropped at that temperature. Subsequently, the same procedures as those in Synthetic Example 7 were applied to obtain 23.3 g (90.5% yield) of the aimed product as white crystals.

Melting point 119.0~120.0 °C

IR (KBr) $cm^{-1}$

1770, 660

| Elementary analysis $C_{22}H_{21}O_3SSbF_6$ | | |
|---|---|---|
| Theoretical value<br>Measured value | C ; 43.95%,<br>C ; 44.03%, | H ; 3.52%<br>H ; 3.83% |

NMR (Acetone-$d_6$) ppm

$\delta$ = 3.50 (3H, S, $\underline{CH_3}S^+$<)

$\delta$ = 5.10 (2H, dd, J = 16Hz, 12Hz, $C_6H_5\underline{CH_2}$-)

$\delta$ = 5.32 (2H, S, $C_6H_5\underline{CH_2}O$-)

$\delta$ = 7.34~8.05 (14H, m, $C_6\underline{H_5}$- × 2, -$C_6\underline{H_4}$-)

Synthetic Example 12

Synthesis for benzyl-4-(benzyloxycarbonyloxy)phenylmethylsulfonium hexafluorophosphate

Benzyl-4-hydroxyphenylmethylsulfonium hexafluorophosphate was dissolved in an amount of 20.0 g (0.053 mol) into 230 ml of acetonitrile, to which 5.7 g (0.056 mol) of triethylamine was added at a temperature lower than 5 °C and 9.6 g (0.056 mol) of benzyl chloroformate was dropped at that temperature. Subsequently, the same procedures as those in Synthetic Example 7 were applied to obtain 23.4 g (86.2% yield) of the aimed product as white crystals.

Melting point 114.0~116.0 °C

IR (KBr) $cm^{-1}$

1750, 830

| Elementary analysis $C_{22}H_{21}O_3SPF_6$ | | |
|---|---|---|
| Theoretical value<br>Measured value | C ; 51.77%,<br>C ; 51.57%, | H ; 4.14%<br>H ; 4.22% |

NMR (Acetone-$d_6$) ppm
δ = 3.50 (3H, S, $\underline{CH_3}S^+$<)
δ = 5.10 (2H, dd, J = 16Hz, 12Hz, $C_6H_5\underline{CH_2}$-)
δ = 5.32 (2H, S, $C_6H_5\underline{CH_2}O$-)
δ = 7.34~8.05 (14H, m, $C_6\underline{H_5}$- × 2, -$C_6\underline{H_4}$-)

Synthetic Example 13

Synthesis for p-methylbenzyl-4-(benzyloxycarbonyloxy)phenylmethylsulfonium hexafluoroantimonate

Benzyl-4-hydroxyphenylmethylsulfonium hexafluoroantimonate was dissolved in an amount of 24.06 g (0.050 mol) into 250 ml of acetonitrile, to which 5.36 g (0.053 mol) of triethylamine was added at a temperature lower than 5°C and 9.04 g (0.053 mol) of benzyl chloroformate was dropped at that temperature. Subsequently, the same procedures as those in Synthetic Example 7 were applied to obtain 27.2 g (88.3% yield) of the aimed product as white crystals.

Melting point 98.5~101.0°C
IR (KBr) $cm^{-1}$
1755, 665

| Elementary analysis $C_{23}H_{23}O_3SSbF_6$ | | |
|---|---|---|
| Theoretical value<br>Measured value | C ; 44.90%,<br>C ; 44.96%, | H ; 3.77%<br>H ; 3.61% |

NMR (Acetone-$d_6$) ppm
δ = 2.32 (3H, S, -$C_6H_4\underline{CH_3}$)
δ = 3.48 (3H, S, $\underline{CH_3}S^+$<)
δ = 5.10 (2H, dd, J = 16Hz, 12Hz, -$\underline{CH_2}C_6H_4$-)
δ = 5.31 (2H, S, $C_6H_5\underline{CH_2}O$-)
δ = 7.18~8.05 (13H, m, $C_6\underline{H_5}$-, -$C_6\underline{H_4}$- × 2)

Synthetic Example 14

Synthesis for benzyl-4-chloroacetoxyphenylmethylsulfonium hexafluoroantimonate

Benzyl-4-hydroxyphenylmethylsulfonium hexafluoroantimonate was dissolved in an amount of 24.0 g (0.051 mol) into 280 ml of acetonitrile, to which 5.46 g (0.054 mol) of triethylamine was added at a temperature lower than 5°C and 6.09 g (0.054 mol) of chloroacetyl chloride was dropped at that temperature. Subsequently, the same procedures as those in Synthetic Example 7 were applied to obtain 24.3 g (87.0% yield) of the aimed product as white crystals.

Melting point 138.0~140.0°C
IR (KBr) $cm^{-1}$
1780, 660

| Elementary analysis $C_{16}H_{16}O_2SClSbF_6$ | | |
|---|---|---|
| Theoretical value<br>Measured value | C ; 35.49%,<br>C ; 35.51%, | H ; 2.77%<br>H ; 2.96% |

NMR (Acetone-$d_6$) ppm
δ = 3.48 (3H, S, $\underline{CH_3}S^+$<)
δ = 4.59 (2H, S, $Cl\underline{CH_2}COO$-)
δ = 5.12 (2H, dd, J = 16Hz, 12Hz, -$\underline{CH_2}C_6H_5$)
δ = 7.25~8.05 (9H, m, -$C_6\underline{H_4}$-, -$C_6\underline{H_5}$)

Synthetic Example 15

Synthesis for α-naphthylmethyl-4-(methoxycarbonyloxy)phenylmethylsulfonium hexafluoroantimonate

Using α-naphthylmethyl-4-hydroxyphenylmethylsulfonium hexafluoroantimonate synthesized in Synthetic Example 3 as the starting material in an amount of 5.00 g (0.015 mol) and synthesis was conducted in the same procedures as those in Synthetic Example 7 to obtain 6.99 g (81.0% yield) of the aimed product as white crystals.

Melting point 125.5~126.0 °C
IR (KBr) $cm^{-1}$
1760, 1210, 660
NMR (Acetone-$d_6$) ppm
$\delta$ = 3.65 (3H, s, $C\underline{H_3}S^{+}$<)
$\delta$ = 3.90 (3H, s, $C\underline{H_3}OCOO$-)
$\delta$ = 5.39~5.92 (2H, dd, -$C\underline{H_2}C_{10}H_7$)
$\delta$ = 7.33~8.32 (11H, m, -$C_6\underline{H_4}$-, -$CH_2C_{10}\underline{H_7}$)

| Elementary analysis $C_{20}H_{19}O_3SSbF_6$ | | |
|---|---|---|
| Theoretical value<br>Measured value | C ; 41.82%,<br>C ; 42.12%, | H ; 3.33%<br>H ; 3.24% |

Synthetic Example 16

Synthesis for α-naphthylmethyl-4-acetoxyphenylmethylsulfonium hexafluoroantimonate

Using α-naphthylmethyl-4-hydroxyphenylmethylsulfonium hexafluoroantimonate synthesized in Synthetic Example 3 as the starting material in an amount of 5.00 g (0.015 mol) and synthesis was conducted in the same procedures as those in Synthetic Example 7 to obtain 6.65 g (79.3% yield) of the aimed product as white crystals.

Melting point 118.0~120.0 °C
IR (KBr) $cm^{-1}$
1775, 1195, 655
NMR (Acetone-$d_6$) ppm
$\delta$ = 2.29 (3H, S, $C\underline{H_3}COO$-)
$\delta$ = 3.60 (3H, S, $C\underline{H_3}S^{+}$<)
$\delta$ = 5.33~5.87 (2H, dd, -$C\underline{H_2}C_{10}H_7$)
$\delta$ = 7.25~8.31 (11H, m, -$C_6\underline{H_4}$-, -$CH_2C_{10}\underline{H_7}$)

| Elementary analysis $C_{20}H_{19}O_2SSbF_6$ | | |
|---|---|---|
| Theoretical value<br>Measured value | C ; 43.02%,<br>C ; 43.19%, | H ; 3.43%<br>H ; 3.47% |

Comparative Example 1

Synthesis for o-nitrobenzyl-4-hydroxyphenylmethylsulfonium hexafluoroantimonate was tried in the same procedures as those in Synthetic Example 1 except for using benzene instead of methanol, but aimed products could not be obtained.

Comparative Example 2

Synthesis for benzoyl-4-(ethoxycarbonyloxy)phenylmethylsulfonium hexafluoroantimonate was tried in the same procedures as those in Synthetic Example 7 except for using methanol instead of acetonitrile but aimed products could not be obtained.

Comparative Example 3

Benzyl-4-(ethoxycarbonyloxy)phenylmethylsulfonium hexafluoroantimonate was synthesized in the same procedures as those in Synthetic Example 7 except for using DMF instead of acetonitrile, but the aimed products were decomposed upon removing DMF and the yield was 3.4 g (14.8% yield).

Working Example 1

α-naphthylmethyl-4-hydroxyphenylmethylsulfonium hexafluoroantimonate was mixed by 0.034 g to 1.0 g of phenyl glycidyl ether. The mixture was deaerated and subjected to bulk polymerization in a sealed tube at a predetermined temperature for one hour and, after the reaction, the conversion ratio was determined based on $^1$H-NMR spectrum. The result is shown below.

| | | | | |
|---|---|---|---|---|
| Temperature (°C) | 30 | 40 | 60 | 80 |
| Conversion ratio (%) | 0 | 8 | 75 | 86 |

Working Example 2

The sulfonium salt used in Working Example 1 was mixed by 0.034 g to 1.0 g of phenyl glycidyl ether, deaerated and sealed in a tube. When photo-induced bulk polymerization was conducted at 10°C by using a 400 W high pressure mercury lamp having main wavelength at 312 nm as a light source and using a rotary photochemical reactor (RH400-10W type) manufactured by Riko Kagaku Sangyo Co., the conversion ratio in two hours was 65%.

Working Example 3

o-nitrobenzyl-4-hydroxyphenylmethylsulfonium hexafluoroantimonate was dissolved in an amount of 1 g into 100 g of Epikote 828 (bisphenol-A epoxy resin, manufactured by Yuka Shell Epoxy Co.), which was coated to a thickness of 0.5 mm on a glass plate. Then, when it was irradiated by sunlight for 5 hrs, the resin was completely gelled.

Working Examples 4 - 12

Blends comprising 100 parts by weight of Epikote 828 (bisphenol-A epoxy resin, manufactured by Yuka Shell Epoxy Co.) and each 3 parts by weight of various sulfonium salts were prepared and each of them was taken in an amount of about 100 mg and gelling time was measured on a hot plate heated to 150°C. The results are shown in Table 1. Further, when the composition prepared in this way was left at 10°C for one month under light shielding, no remarkable increase of viscosity was observed.

Working Examples 13 - 20

Blends comprising 100 parts by weight of Epikote 828 (bisphenol-A epoxy resin, manufactured by Yuka Shell Epoxy Co.) and each 3 parts by weight of various sulfonium salts were prepared and each of them was taken in an amount of about 100 mg and gelling time was measured on a hot plate heated to 100°C. The results are shown in Table 2.

Working Example 21

Benzyl-4-(benzyloxycarbonyloxy)phenylmethylsulfonium hexafluoroantimonate and 4-(benzoyloxy)-phenylmethylsulfonium hexafluoroantimonate were mixed by 0.040 g and 0.038 g respectively to 1.0 g of phenyl glycidyl ether. The mixture was deaerated and subjected to bulk polymerization in a sealed tube at 50°C for one hour to determine the conversion ratio from $^1$H-NMR spectrum. The results were 75% and 68% respectively.

Working Examples 22 - 29

Various sulfonium salts were mixed each by 0.030 g to 1.0 g of phenyl glycidyl ether. The mixture was deaerated, sealed in a tube and subjected to polymerization at 30 ° C and 60 ° C for one hour respectively, to determine a conversion ratio from $^1$H-NMR spectrum. The results are shown in Table 3. When the compositions thus prepared were left at 5 ° C for one month, no remarkable viscosity increase was observed.

Working Example 30 - 36

Various sulfonium salts were mixed each by 0.030 g to 1.0 g of phenyl glycidyl ether. The mixture was deaerated, sealed in a tube and subjected to photo-induced bulk polymerization for 90 min to determine a conversion ratio from $^1$H-NMR spectrum. A 400 W high pressure mercury lamp was used as a light source and a rotary photochemical reactor manufactured by Riko Kagaku Sangyo was used. The temperature for the polymerization was 10 ° C (under water cooling). The results are shown in Table 4.

Working Examples 37 - 38

Procedures were according to the methods of Working Examples 30 - 36 except for setting the polymerization temperature to 20 ° C. The results are shown in Table 5.

Working Example 39

P-nitrobenzyl-4-hydroxyphenylmethylsulfonium hexafluoroantimonate was mixed in an amount of 1 g into 100 g of Epikote 828 (bisphenol-A epoxy resin, manufactured by Yuka Shell Epoxy Co.), which was coated to a thickness of 0.5 mm on a glass plate. Then, it was irradiated by an ultra-violet lamp (80 W/cm, high pressure mercury lamp) at a distance of 10 cm for 1 min. After irradiation, the resin was completely gelled.

When the mixture was irradiated by a ultra-violet ray lamp for 30 sec., the viscosity was increased. When it was further treated at 120 ° C for 5 min, a glass-like curing product was obtained. When the mixture prepared was left at 15 ° C for one month under light shielding, no viscosity increase was observed.

Working Example 40

p-nitrobenzyl-4-hydroxyphenylmethylsulfonium hexafluoroantimonate was mixed by 0.030 g to 1.0 g of phenyl glycidyl ether. The mixture was deaerated, sealed in a tube and subjected to polymerization at 60 ° C and 120 ° C for one hour respectively, to determine a phenyl glycidyl conversion ratio by using $^1$H-NMR spectrum. The result was 0% at 60 ° C and 70% at 120 ° C. When the thus prepared composition prepared was left at 15 ° C for one month under light shielding, no further remarkable viscosity increase was observed.

Working Example 41

α-naphthylmethyl-4-hydroxyphenylmethylsulfonium hexafluoroantimonate was mixed by 0.015 g to 0.5 g of 1-phenyl-4-ethyl-2,6,7-trioxabicyclo(2,2,2)octane, deaerated and sealed in a tube. The mixture was subjected to bulk polymerization at 120 ° C for 20 hrs. After the polymerization, the conversion ratio was confirmed to be 100% from $^1$H-NMR spectrum. Then, the polymer was dissolved in 2 ml of methylene chloride and poured into 100 ml of hexane. Then, a hexane insoluble portion was dried to obtain 0.35 g of polymer.

Working Example 42

4-(benzoyloxy)phenylmethylsulfonium hexafluoroantimonate was mixed by 0.039 g to 0.5 g of the monomer used in Working Example 41, deaerated and sealed in a tube. The mixture was subjected to bulk polymerization at 80 ° C for 24 hrs.

After the polymerization, the conversion ratio was confirmed to be 100% from $^1$H-NMR spectrum. Then, the polymer was dissolved in 2 ml of methylene chloride and poured into 100 ml of hexane. Then a hexane insoluble portion was dried to obtain 0.40 g of a polymer.

Working Example 43

The sulfonium salt used in Working Example 42 was mixed by 0.025 g to 0.5 g of 3,9-dibenzyl-1,5,7,11-tetraoxaspiro(5,5)undecane, deaerated and sealed in a tube. The mixture was subjected to bulk polymerization at 80 °C for 2 hrs. After the polymerization, the conversion ratio was confirmed to be 99% from $^1$H-NMR spectrum. Then, the polymer was dissolved in 2 ml of methylene chloride and poured into 100 ml of hexane. Then, a hexane insoluble portion was dried to obtain 0.45 g of a polymer.

Working Example 44

o-nitrobenzyl-4-hydroxyphenylmethylsulfonium hexafluoroantimonate was mixed by 0.015 g to 0.5 g of the monomer in Working Example 43, deaerated and sealed in a tube. The mixture was subjected to bulk polymerization at 150 °C for 2 hours. After the polymerization, the conversion ratio was confirmed to be 95% from $^1$H-NMR spectrum. Then, the polymer was dissolved in 2 ml of methylene chloride and poured into 100 ml of hexane. Then, a hexane insoluble portion was dried to obtain 0.40 g of a polymer.

Working Example 45

o-nitrobenzyl-4-hydroxyphenylmethylsulfonium hexafluoroantimonate was mixed by 0.010 g to 0.5 g of purified styrene, deaerated and sealed in a tube. Then, polymerization was taken place under stirring at 100 °C. After 60 min, the solidified reaction solution was dissolved by 2 ml of carbon tetrachloride and the conversion ratio was confirmed to be 89% from $^1$H-NMR spectrum. The product was then poured into methanol to stop the polymerization and deposit the polymer. The purified and dried polymer was white crystals and the yield was 75%. Further, based on GPC (converted as polystyrene), $\overline{Mn}$ = 3800, $\overline{Mw}/\overline{Mn}$ = 1.89.

Working Examples 46 - 53

Styrene was polymerized by using various sulfonium salts according to Working Examples 45. The results are shown in Table 6.

Working Example 54

Styrene was polymerized at 60 °C for 60 min with the same formulation as Working Example 45, but the conversion ratio was 0%.

Further, 10 g of purified styrene and 0.30 g of the sulfonium salt used in Working Example 45 were dissolved in 20 ml of chlorobenzene, deaerated and sealed in a tube. When they were left at 10 °C for one month under light shielding, polymerization of styrene did not occur.

Working Example 55

Benzyl-4-(benzyloxycarbonyloxy)phenylmethylsulfonium hexafluoroantimonate was mixed by 0.030 g to 0.5 g of purified styrene, deaerated and sealed in a tube. Then, polymerifzation was taken place under stirring at 60 °C.

Subsequently, they were treated in the same procedures as thoes in Working Example 45. The conversion ratio was confirmed to be 97%. The polymer was white crystals and the yield was 85%. Further, based on GPC (converted as polystyrene), $\overline{Mn}$ = 4200, $\overline{Mw}/\overline{Mn}$ = 2.01.

Working Examples 56 - 61

Styrene was polymerized by using various sulfonium salts according to Working Example 55. The results are shown in Table 7.

Working Example 62

Solution polymerization was conducted by using 2 ml of chlorobenzene as a solvent under the conditions as in Working Example 55 to obtain polystyrene. Conversion rate: 99%, yield: 85%, $\overline{Mn}$ = 3900, $\overline{Mw}/\overline{Mn}$ = 1.86.

Styrene was polymerized at 10 °C for 60 min according to Working Example 55, but the conversion ratio was 0%.

Working Example 63

Polymerization was conducted quite in the same manner as in Working Example 43 except for using 0.5 g of n-butyl vinyl ether instead of 0.5 g of styrene, to obtain a polymer of n-butyl vinyl ether. Conversion ratio : 90%, yield: 71%, $\overline{Mn}$ = 4700, $\overline{Mw}/\overline{Mn}$ = 1.91.

Working Example 64

10 g of purified styrene and 1.73 g of benzyl-4-(benzyloxycarbonyloxy)phenylmethylsulfonium hexafluoroantimonate were dissolved in 20 ml of chlorobenzene, deaerated and sealed in a tube. Then, when they were left at 5 °C for one month, polymerization of styrene did not occur.

Working Example 65

p-chlorobenzyl-4-hydroxyphenylmethylsulfonium hexafluoroantimonate was mixed by 0.015 g to 0.5 g of 1-phenyl-4-ethyl-2,6,7-trioxabicyclo(2,2,2)octane, deaerated and sealed in a tube. The mixture was subjected to bulk polymerization at 120 °C for 24 hrs. After the polymerization, the conversion ratio was confirmed to be 100% from $^1$H-NMR spectrum. Then, the polymer was dissolved in 2 ml of methylene chloride and poured into 100 ml of hexane. Then, a hexane insoluble portion was dried to obtain 0.33 g of a polymer.

Working Example 66

p-chlorobenzyl-4-hydroxyphenylmethylsulfonium hexafluoroantimonate was mixed by 0.015 g to 0.5 g of 3,9-dibenzyl-1,5,7,11-tetraoxaspiro(5,5)undecane, deaerated and sealed in a tube. The mixture was subjected to bulk polymerization at 130 °C for 2 hrs. After the polymerization, the conversion ratio was confirmed to be 96% from $^1$H-NMR spectrum.

Then, the polymer was dissolved in 2 ml of methylene chloride and poured into 100 ml of hexane. Then a hexane insoluble portion was dried to obtain 0.43 g of a polymer.

Working Examples 67 - 68

Compositions comprising each 100 parts by weight of Epikote 828 (bisphenol-A epoxy resin, manufactured by Yuka Shell Epoxy) and 3 parts by weight of various sulfonium salts were prepared. Then, each of the compositions was charged in a glass bottle and heated in an oil bath to conduct a curing test at 150 °C and measure the time required for the curing. The results are shown in Table 8.

Further, when each of the compositions was left at 15 °C for one month, no remarkable viscosity increase was observed. Further, when each of the compositions of was left at 15 °C for one month under light sheilding, no remarkable viscosity increase was observed.

Table 1

| Working Example | Sulfomium salt | Gelling time |
|---|---|---|
| 4 | 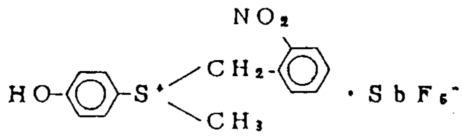 | 1 min |
| 5 | 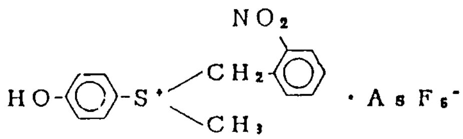 | 1 min 30 sec |
| 6 | 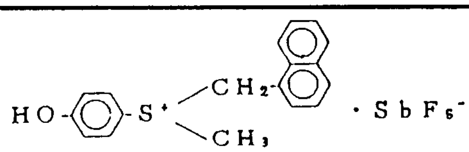 | 5 sec |
| 7 | 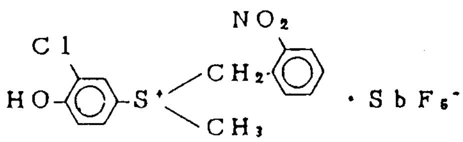 | 45 sec |
| 8 | 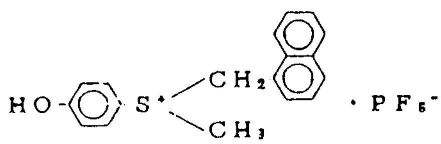 | 2 min |
| 9 | 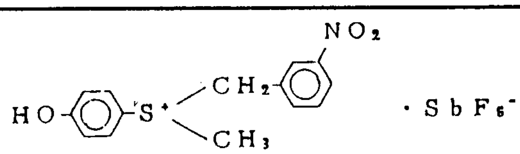 | 1 min 45 sec |
| 10 | 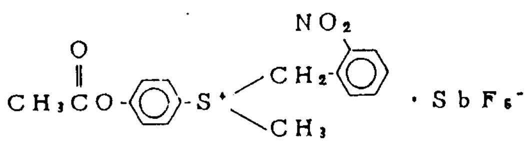 | 10 sec |
| 11 | 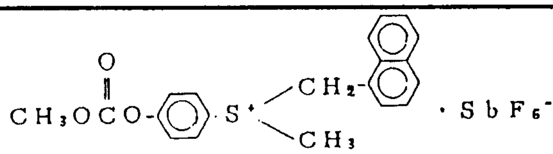 | <1 sec |
| 12 | 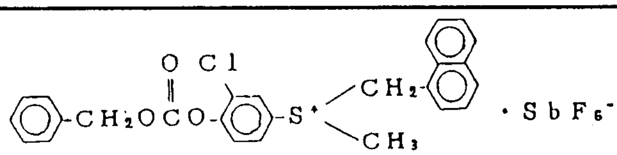 | 10 sec |
| Comparison | 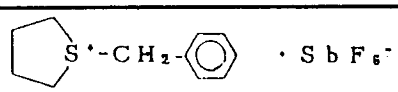 | 4 min 30 sec |

Table 2

| Working Example | Benzyl sulfonium salt | Gelling time |
|---|---|---|
| 13 | 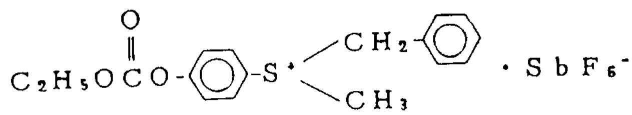 | 15 sec |
| 14 | 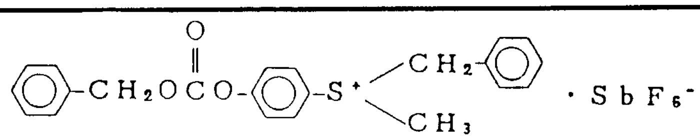 | 12 sec |
| 15 | 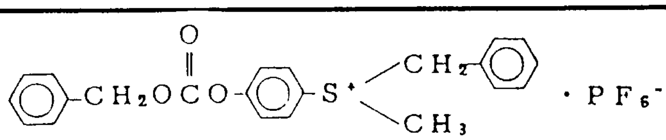 | 2 min 10 sec |
| 16 | 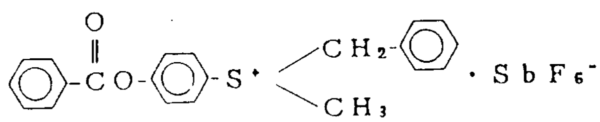 | 13 sec |
| 17 | 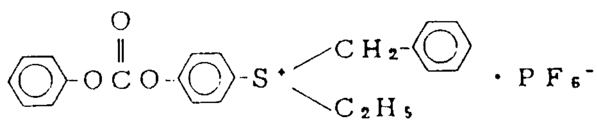 | 1 min 50 sec |
| 18 | 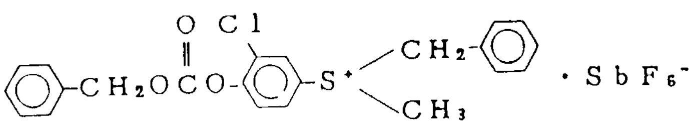 | 6 sec |
| 19 | 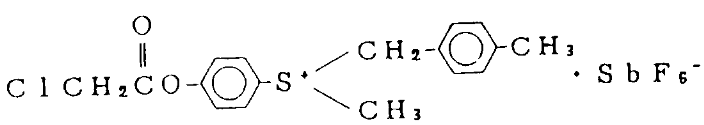 | 1 sec |
| 20 | 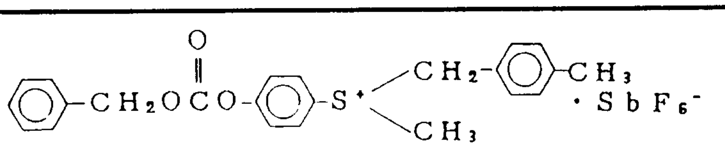 | <1 sec |
| Comparison | 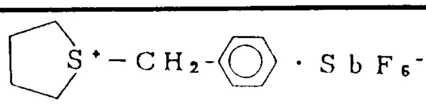 | > 30 min |
| Comparison | 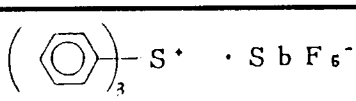 | > 60 min |

Table 3

| Working Example | Benzyl sulfonium salt | Conversion ratio (%) | |
|---|---|---|---|
| | | 30°C | 60°C |
| 22 | 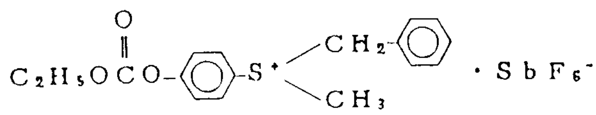 | 10 | 75 |
| 23 | 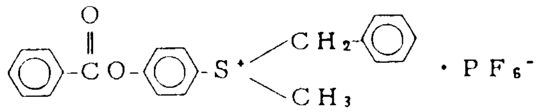 | 2 | 72 |
| 24 | 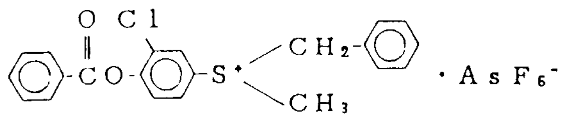 | 0 | 69 |
| 25 | 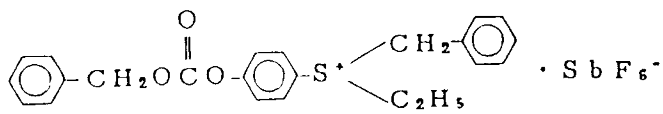 | 12 | 79 |
| 26 | 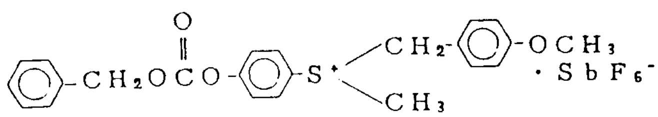 | 52 | 90 |
| 27 | 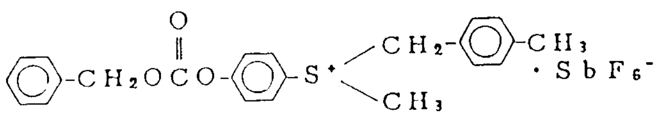 | 41 | 85 |
| 28 | 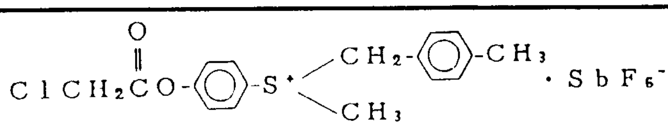 | 40 | 86 |
| 29 | 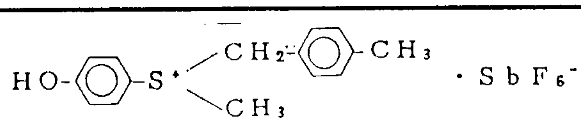 | 9 | 51 |
| Comparative Example | 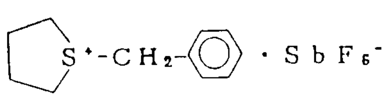 | 0 | 1 |

Table 4

| Working Example | Benzyl sulfonium salt | Conversion ratio (%) |
|---|---|---|
| 30 | 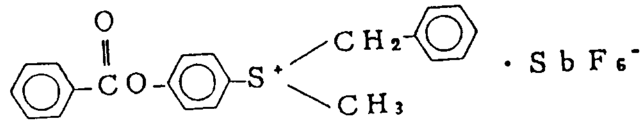 | 70 |
| 31 | 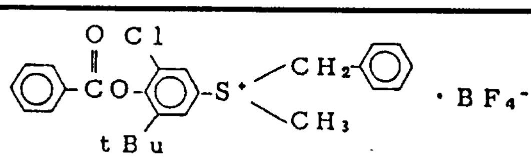 | 59 |
| 32 | 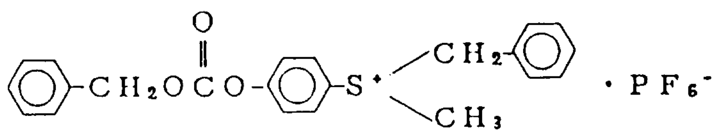 | 60 |
| 33 | 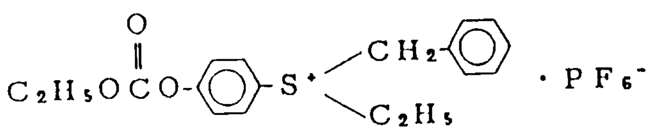 | 65 |
| 34 | 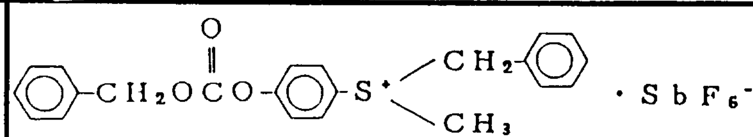 | 42 |
| 35 | 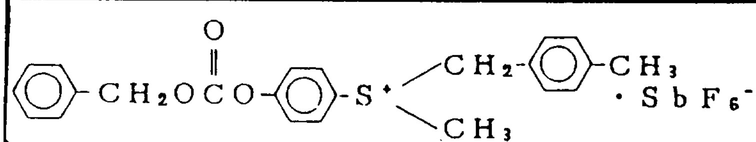 | 56 |
| 36 | 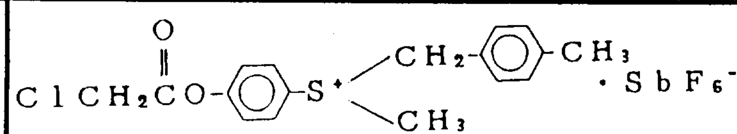 | 55 |
| Comparative Example 1 | 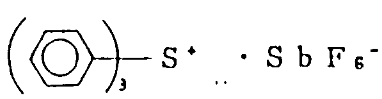 | 59 |
| Comparative Example 2 | 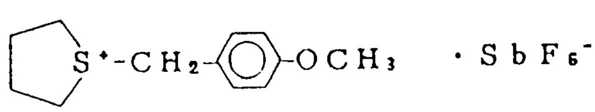 | 10 |

Table 5

| Working Example | Substituted benzyl sulfonium salt | Conversion ratio (%) |
|---|---|---|
| 37 | 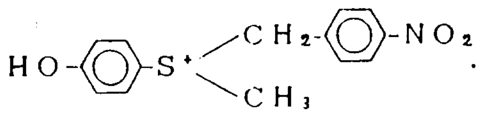 $\cdot SbF_6^-$ | 69 |
| 38 | 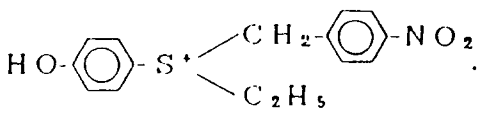 $\cdot SbF_6^-$ | 66 |
| Comparative Example 1 | 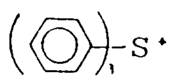 $\cdot SbF_6^-$ | 59 |
| Comparative Example 2 | 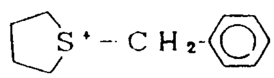 $\cdot SbF_6^-$ | 0 |

Table 6

| Working Example | Sulfonium salt | Conversion ratio (%) | Yield (%) | Mn | Mw/Mn |
|---|---|---|---|---|---|
| 46 | 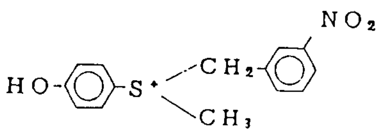 · $SbF_6^-$ | 89 | 78 | 3900 | 2.00 |
| 47 | 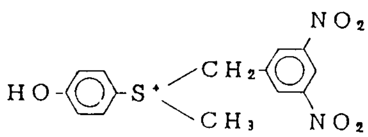 · $SbF_6^-$ | 85 | 71 | 4000 | 1.90 |
| 48 | 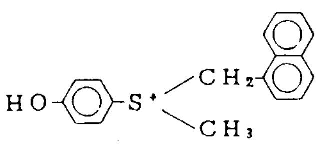 · $SbF_6^-$ | 96 | 84 | 3800 | 2.05 |
| 49 | 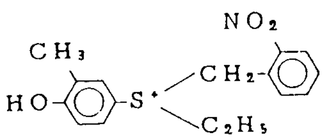 · $SbF_6^-$ | 86 | 74 | 4200 | 1.87 |
| 50 | 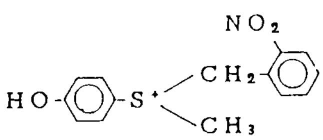 · $PF_6^-$ | 71 | 59 | 3900 | 1.80 |
| 51 | 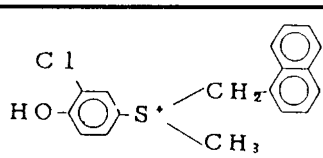 · $SbF_6^-$ | 99 | 88 | 3700 | 2.32 |
| 52 | 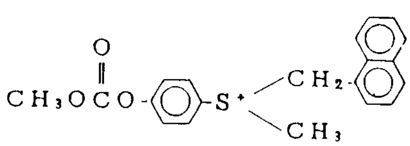 · $SbF_6^-$ | 100 | 90 | 3600 | 2.35 |
| 53 | 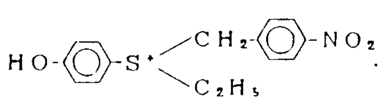 · $AsF_6^-$ | 97 | 83 | 4400 | 2.01 |

Table 7

| Working Example | Benzyl sulfonium salt | Conversion ratio (%) | Yield (%) | Mn | Mw/Mn |
|---|---|---|---|---|---|
| 56 | 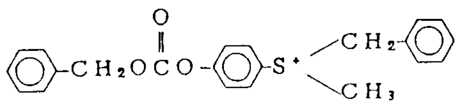 $\cdot SbF_6^-$ | 97 | 84 | 4100 | 2.02 |
| 57 | 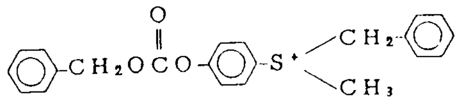 $\cdot BF_4^-$ | 70 | 57 | 2600 | 1.76 |
| 58 | 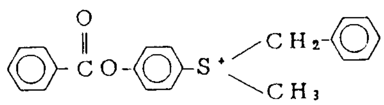 $\cdot PF_6^-$ | 85 | 76 | 3400 | 1.91 |
| 59 | 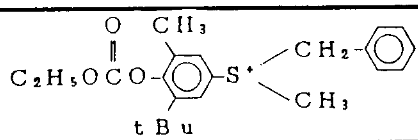 $\cdot AsF_6^-$ | 78 | 70 | 2800 | 1.70 |
| 60 | 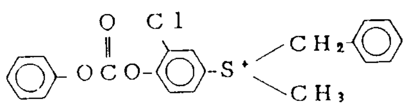 $\cdot SbF_6^-$ | 98 | 83 | 4200 | 1.80 |
| 61 | 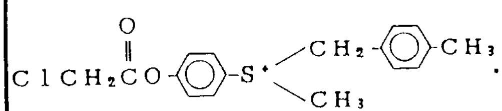 $\cdot SbF_6^-$ | 98 | 85 | 4100 | 2.00 |

Table 8

| Working Example | Substituted benzyl sulfomium salt | Gelling time |
|---|---|---|
| 69 | 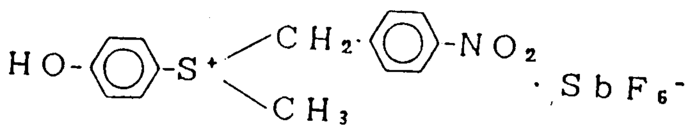 | 2 min 30 sec |
| 70 | 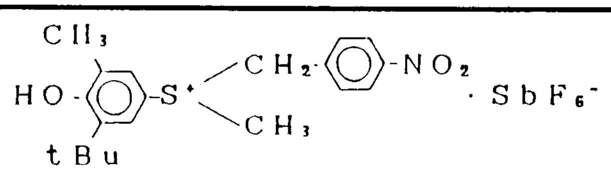 | 2 min 40 sec |
| Comparative Example | 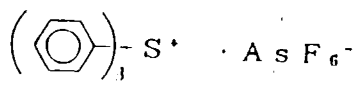 | >60 min |

(Effect of the Invention)

As apparent from the synthetic examples and working examples described above, the novel sulfonium compound according to the present invention is effective as an epoxy curing agent requiring high purity and, further, as industrial intermediate material.

Further, the specified sulfonium salt according to the present invention is useful as the polymerization initiator for cationically polymerizable material, and the polymer composition containing the same can be polymerized and cured by the polymerization process based on the irradiation of radiation rays such as light and electron beam and/or by heat treatment. In particular, the sulfonium salt of the general formula (II) as defined in claim 5, wherein $R_2 = R_3 = H$, $R_5 = CH_3$, $R_6$ = ethoxycarbonyl group or benzyloxycarbonyl group and Q = methylbenzyl group shows high activity to heat and a one-component type composition prepared therefrom is polymerizable or curable rapidly at a room temperature or under slight heating, although it should be stored at low temperature. Further, the sulfonium salt of the general formula (II) as defined in claim 5, wherein $R_2 = R_3 = H$, $R_5 = CH_3$, $R_6 = H$ and Q = nitrobenzyl group shows high activity to light and polymerizable or curable only by irradiating radiation rays for a short time and it has excellent storability under light shielding and at a room temperature.

**Claims**

**1.** A sulfonium compound represented by the following general formula (I):

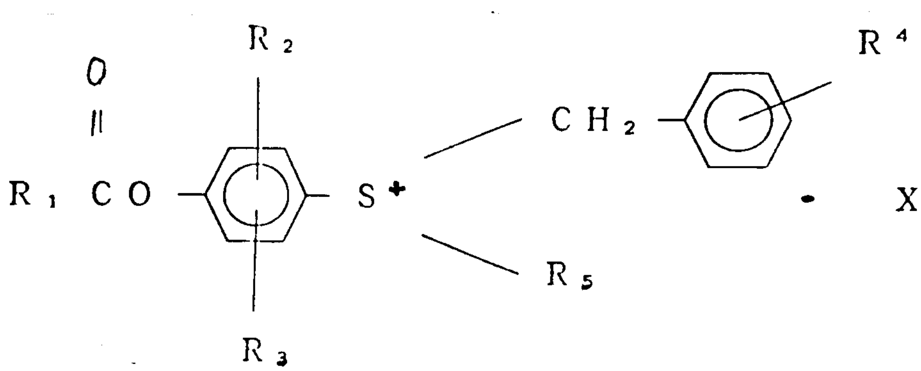

...... ( I )

where $R_1$ represents ethoxy group, phenyl group, benzyloxy group, phenoxy group or 9-fluroenylmethoxy group, $R_2$ and $R_3$ represent, independently, hydrogen, halogen or $C_1$ - $C_4$ alkyl group, $R_4$ represents hydrogen or methoxy group, $R_5$ represents $C_1$ - $C_4$ alkyl group and $X^-$ represents $SbF_6$, $PF_6$, $AsF_6$ or $BF_4$.

**2.** A sulfonium compound of claim 1, wherein the sulfonium compound is benzyl-4-(benzyloxycarbonyloxy)phenylmethylsulfonium hexafluoroamtinonate.

**3.** A sulfonium compound of claim 1, wherein the sulfonium compound is benzyl-4-(ethoxycarbonyloxy)-phenylmethylsulfonium hexafluoroamtinonate.

**4.** A sulfonium compound of claim 1, wherein the sulfonium compound is benzyl-4-(phenoxycarbonyloxy)-phenylmethylsulfonium.

**5.** A sulfonium compound represented by the following general formula (II) :

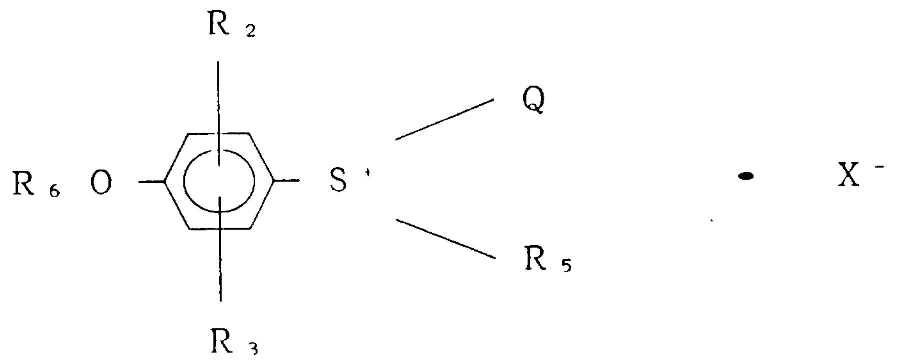

... (II)

where $R_2$ and $R_3$ represent, independently, hydrogen, halogen or $C_1$ - $C_4$ alkyl group, $R_5$ represents $C_1$ - $C_4$ alkyl group, $R_6$ represents hydrogen, methyl group, acetyl group, methoxycarbonyl group, ethoxycarbonyl group, chloroacetyl group, trichloroacetyl group, benzyloxy carbonyl group, benzoyl group, phenoxycarbonyl group or 9-fluorenylmethoxycarbonyl group, Q represents chlorobenzyl group, methylbenzyl group, nitrobenzyl group, dinitrobenzyl group, $\alpha$-naphthylmethyl group or $\beta$-naphthylmethyl group and X represents $SBF_6$, $PF_6$, $AsF_6$ or $BF_4$, providing that when $R_6$ is hydrogen, methyl group, acetyl group or methoxycarbonyl group, Q does not include chlorobenzyl group or methylbenzyl group.

**6.** A sulfonium compound of claim 5 wherein the sulfonium compound is p-methylbenzyl-4-(benzyloxycarbonyloxy) phenylmethylsulfonium hexafluoroantimonate.

**7.** A sulfonium compound of claim 5 wherein the sulfonium compound is p-methylbenzyl-4-(benzyloxycarbonyloxy) phenylmethylsulfonium hexafluorophosphate.

**8.** A sulfonium compound of claim 5 wherein the sulfonium compound is p-nitrobenzyl-4-hydroxyphenylmethylsulfonium hexafluoroantimonate.

**9.** A sulfonium compound of claim 5 wherein the sulfonium compound is p-nitrobenzyl-4-hydroxyphenylmethylsulfonium hexafluorophosphate.

**10.** A sulfonium compound of claim 5 wherein the sulfonium compound is $\alpha$-naphthylmethyl-4-hydroxyphenylmethylsulfonium hexafluoroantimonate.

**11.** A sulfonium compound of claim 5 wherein the sulfonium compound is $\alpha$-naphthylmethyl-4-hydroxyphenylmethylsulfonium hexafluorophosphate.

**12.** A sulfonium compound of claim 5 wherein the sulfonium compound is $\alpha$-naphthylmethyl-4-(metoxycarbonyloxy)phenylmethylsulfonium hexafluoroantimonate.

**13.** A sulfonium compound of claim 5 wherein the sulfonium compound is α-naphthylmethyl-4-(metoxycarbonyloxy)phenylmethylsulfonium hexafluorophosphate.

**14.** A sulfonium compound of claim 5 wherein the sulfonium compound is α-naphthylmethyl-4-(benzyloxycarbonyloxy)phenylmethylsulfonium hexafluoroantimonate.

**15.** A sulfonium compound of claim 5 wherein the sulfonium compound is α-naphthylmethyl-4-(benzyloxycarbonyloxy)phenylmethylsulfonium hexafluorophosphate.

**16.** A sulfonium compound of claim 1 wherein the sulfonium compound is benzyl-4-(benzyloxycarbonyloxy)phenylmethylsulfonium hexafluoroantimonate.

**17.** A polymerization initiator for cationically polymerizable material comprising a sulfonium salt represented by the general formula (I) as defined in claim 1 and/or by the general formula (II) as defined in claim 5.

**18.** A polymerization initiator as defined in claim 17, wherein sulfonium compound is benzyl-4-(benzyloxycarbonyloxy)phenylmethylsulfonium hexafluoroantimonate.

**19.** A polymerization initiator as defined in claim 17, wherein sulfonium compound is benzyl-4-(ethoxycarbonyloxy)phenylmethylsulfoniumhexafluoroantimonate.

**20.** A polymerization initiator as defined in claim 17, wherein sulfonium compound is benzyl-4-(phenoxycarbonyloxy)phenylmethylsulfonium hexafluoroantimonate.

**21.** A polymerization initiator as defined in claim 17, wherein sulfonium compound is p-methylbenzyl-4-(benzyloxycarbonyloxy)phenylmethylsulfonium hexafluoroantimonate.

**22.** A polymerization initiator as defined in claim 17, wherein sulfonium compound is p-methylbenzyl-4-(benzyloxycarbonyloxy)phenylmethylsulfonium hexafluorophosphate.

**23.** A polymerization initiator as defined in claim 17, wherein sulfonium compound is p-nitrobenzyl-4-hydroxyphenylmethylsulfonium hexafluoroantimonate.

**24.** A polymerization initiator as defined in claim 17, wherein sulfonium compound is p-nitrobenzyl-4-hydroxyphenylmethylsulfonium hexafluorophosphate.

**25.** A polymerization initiator as defined in claim 17, wherein sulfonium compound is α-naphthylmethyl-4-hydroxyphenylmethylsulfonium hexafluoroantimonate.

**26.** A polymerization initiator as defined in claim 17, wherein sulfonium compound is α-naphthylmethyl-4-hydroxyphenylmethylsulfonium hexafluorophosphate.

**27.** A polymerization initiator as defined in claim 18, wherein sulfonium compound is α-naphthylmethyl-4-(methoxycarbonyloxy)phenylmethylsulfonium hexafluoroantimonate.

**28.** A polymerization initiator as defined in claim 17, wherein sulfonium compound is α-naphthylmethyl-4-(methoxycarbonyloxy)phenylmethylsulfonium hexafluorophosphate.

**29.** A polymerization initiator as defined in claim 17, wherein sulfonium compound is α-naphthylmethyl-4-(benzyloxycarbonyloxy)phenylmethylsulfonium tetrafluoroantimonate.

**30.** A polymerization initiator as defined in claim 17, wherein sulfonium compound is α-naphthylmethyl-4-(benzyloxycarbonyloxy)phenylmethylsulfonium hexafluorophosphate.

**31.** A process for polymerizing cationically polymerizable material, which comprises adding one or more of sulfonium salt represented by the general formula (I) as defined in claim 1 and/or by the general formula (II) as defined in claim 5 as a initiator to one or more of cationically polymerizable material and polymerizing them by means of radiation beam and/or heat.

**32.** A process as defined in claim 31, wherein the cationically polymerizable material is a compound having an epoxy group.

**33.** A process as defined in claim 31, wherein the cationically polymerizable material is a cationically polymerizable vinyl compound.

**34.** A process as defined in claim 31, wherein the cationically polymerizable material is a compound having one or more of spiro-ortho ester group, bicyclo-ortho ester group or spiro-ortho carbonate group.

**35.** A process as defined in claim 31, wherein the radiation rays are ultraviolet rays.

**36.** A process as defined in claim 31, wherein the polymerization is conducted at a temperature higher than 20 °C.

**37.** A process as defined in claim 31, wherein the radiation rays are sunlight.

**38.** A process as defined in claim 31, wherein from 0.01 to 20 parts by weight of a sulfonium salt represented by the general formula (I) as defined in claim 1 and/or by the general Formula (II) as defined in claim 5 is added to 100 parts by weight of the cationically polymerizable material.

**Patentansprüche**

**1.** Sulfoniumverbindung gemäß der folgenden allgemeinen Formel (I):

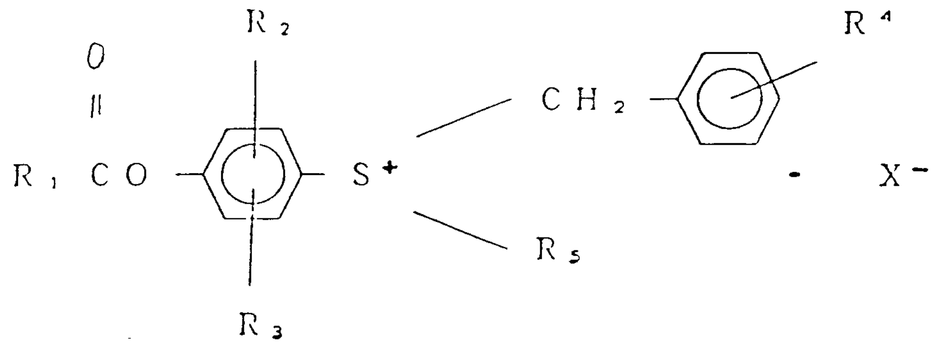

...... ( I )

wobei

| | |
|---|---|
| $R_1$ | eine Ethoxy-Gruppe, Phenyl-Gruppe, Benzyloxy-Gruppe, Phenoxy-Gruppe oder 9-Fluorenylmethoxy-Gruppe, |
| $R_2$ und $R_3$ | unabhängig voneinander Wasserstoff, ein Halogen oder eine $C_1$ - $C_4$ Alkyl-Gruppe, |
| $R_4$ | Wasserstoff oder eine Methoxy-Gruppe, |
| $R_5$ | eine $C_1$ - $C_4$ Alkyl-Gruppe, und |
| $X^-$ | ein Fluorid der Zusammensetzung $SbF_6$, $PF_6$, $AsF_6$ or $BF_4$ |

darstellen.

**2.** Sulfoniumverbindung nach Anspruch 1, **dadurch gekennzeichnet**, daß als Sulfoniumverbindung Benzyl-4-(Benzyloxykarbonyloxy)-Phenylmethylsulfonium-Hexafluoroantimonat vorgesehen ist.

**3.** Sulfoniumverbindung nach Anspruch 1, **dadurch gekennzeichnet**, daß als Sulfoniumverbindung Benzyl-4-(Ethoxykarbonyloxy)-Phenylmethylsulfonium - Hexafluoroantimonat vorgesehen ist.

**4.** Sulfoniumverbindung nach Anspruch 1, **dadurch gekennzeichnet**, daß als Sulfoniumverbindung Benzyl-4-(Phenoxykarbonyloxy)-Phenylmethylsulfonium vorgesehen ist.

**5.** Sulfoniumverbindung gemäß der folgenden allgemeinen Formel II:

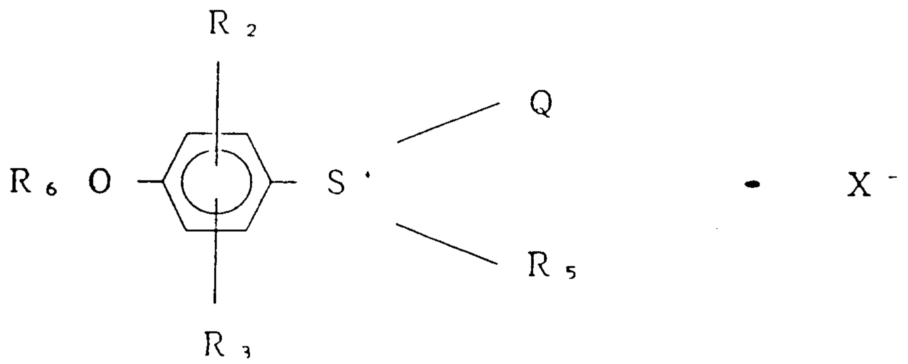

... (II)

wobei

| | |
|---|---|
| $R_2$ und $R_3$ | unabhängig voneinander Wasserstoff, ein Halogen oder eine $C_1$ - $C_4$ Alkyl-Gruppe, |
| $R_5$ | eine $C_1$ - $C_4$ Akyl-Gruppe, |
| $R_6$ | Wasserstoff, eine Methyl-Gruppe, Azetyl-Gruppe, Methoxykarbonyl-Gruppe, Ethoxykarbonyl-Gruppe, Chloroazetyl-Gruppe, Trichloroazetyl-Gruppe, Benzyloxykarbonyl-Gruppe, Benzoyl-Gruppe, Phenoxykarbonyl-Gruppe oder 9-Fluorenylmethoxykarbonyl-Gruppe, |
| Q | eine Chlorobenzyl-Gruppe, Methylbenzyl-Gruppe, Nitrobenzyl-Gruppe, Dinitrobenzyl-Gruppe, alpha-Naphtylmetyl-Gruppe oder beta-Naphtylmethyl-Gruppe, und |
| X | ein Fluorid der Zusammensetzung $SbF_6$, $PF_6$, $AsF_6$ or $BF_4$ |

darstellen, vorausgesetzt, daß dann, wenn $R_6$ Wasserstoff, eine Methyl-Gruppe, Azetyl-Gruppe oder eine Methoxykarbonyl-Gruppe ist, Q keine Chlorobenzyl-Gruppe oder Methylbenzyl-Gruppe enthält.

**6.** Sulfoniumverbindung nach Anspruch 5, **dadurch gekennzeichnet**, daß als Sulfoniumverbindung p-Methylbenzyl-4-(Benzyloxykarbonyloxy)-Phenylmethylsulfonium-Hexafluoroantimonat vorgesehen ist.

**7.** Sulfoniumverbindung nach Anspruch 5, **dadurch gekennzeichnet**, daß als Sulfoniumverbindung p-Methylbenzyl-4-(Benzyloxykarbonyloxy)-Phenylmethylsulfonium-Hexafluorophosphat vorgesehen ist.

**8.** Sulfoniumverbindung nach Anspruch 5, **dadurch gekennzeichnet**, daß als Sulfoniumverbindung p-Nitrobenzyl-4-Hydroxyphenylmethylsulfonium-Hexafluoroantimonat vorgesehen ist.

**9.** Sulfoniumverbindung nach Anspruch 5, **dadurch gekennzeichnet**, daß als Sulfoniumverbindung p-Nitrobenzyl-4-Hydroxyphenylmethylsulfonium-Hexafluorophosphat vorgesehen ist.

**10.** Sulfoniumverbindung nach Anspruch 5, **dadurch gekennzeichnet**, daß als Sulfoniumverbindung alpha-Naphtylmethyl-4-Hydroxyphenylmethylsulfonium-Hexafluoroantimonat vorgesehen ist.

**11.** Sulfoniumverbindung nach Anspruch 5, **dadurch gekennzeichnet**, daß als Sulfoniumverbindung alpha-Naphtylmethyl-4-Hydroxyphenylmethylsulfonium-Hexafluorophosphat vorgesehen ist.

**12.** Sulfoniumverbindung nach Anspruch 5, **dadurch gekennzeichnet**, daß als Sulfoniumverbindung alpha-Naphtylmethyl-4-(Metoxykarbonyloxy)-Phenylmethylsulfonium-Hexafluoroantimonat vorgesehen ist.

**13.** Sulfoniumverbindung nach Anspruch 5, **dadurch gekennzeichnet**, daß als Sulfoniumverbindung alpha-Naphtylmethyl-4-(Metoxykarbonyloxy)-Phenylmethylsulfonium-Hexafluorophosphat vorgesehen ist.

**14.** Sulfoniumverbindung nach Anspruch 5, **dadurch gekennzeichnet**, daß als Sulfoniumverbindung alpha-Naphtylmethyl-4-(Benzyloxykarbonyloxy)-Phenylmethylsulfonium-Hexafluoroantimonat vorgesehen ist.

**15.** Sulfoniumverbindung nach Anspruch 5, **dadurch gekennzeichnet**, daß als Sulfoniumverbindung alpha-Naphtylmethyl-4-(Benzyloxykarbonyloxy)-Phenylmethylsulfonium-Hexafluorophosphat vorgesehen ist.

**16.** Sulfoniumverbindung nach Anspruch 1, **dadurch gekennzeichnet**, daß als Sulfoniumverbindung Benzyl-4-(Benzyloxykarbonyloxy)-Phenylmethylsulfonium-Hexafluoroantimonat vorgesehen ist.

**17.** Polymerisierungsinitiator für einen kationisch polymersierbaren Stoff, mit einem Sulfoniumsalz gemäß der in Anspruch 1 angegebenen allgemeinen Formel (I) und/oder der in Anspruch 5 angegebenen allgemeinen Formel (II).

**18.** Polymerisierungsinitiator nach Anspruch 17, **dadurch gekennzeichnet**, daß als Sulfoniumverbindung Benzyl-4-(Benzyloxykarbonyloxy)-Phenylmethylsulfonium-Hexafluoroantimonat vorgesehen ist.

**19.** Polymerisierungsinitiator nach Anspruch 17, **dadurch gekennzeichnet**, daß als Sulfoniumverbindung Benzyl-4-(Ethoxykarbonyloxy)-Phenylmethylsulfonium-Hexafluoroantimonat vorgesehen ist.

**20.** Polymerisierungsinitiator nach Anspruch 17, **dadurch gekennzeichnet**, daß als Sulfoniumverbindung Benzyl-4-(Phenoxykarbonyloxy)-Phenylmethylsulfonium-Hexafluoroantimonat vorgesehen ist.

**21.** Polymerisierungsinitiator nach Anspruch 17, **dadurch gekennzeichnet**, daß als Sulfoniumverbindung p-Methylbenzyl-4-(Benzyloxykarbonyloxy)-Phenylmethylsulfonium-Hexafluoroantimonat vorgesehen ist.

**22.** Polymerisierungsinitiator nach Anspruch 17, **dadurch gekennzeichnet**, daß als Sulfoniumverbindung p-Methylbenzyl-4-(Benzyloxykarbonyloxy)-Phenylmethylsulfonium-Hexafluorophosphat vorgesehen ist.

**23.** Polymerisierungsinitiator nach Anspruch 17, **dadurch gekennzeichnet**, daß als Sulfoniumverbindung p-Nitrobenzyl-4-Hydroxy-Phenylmethylsulfonium-Hexafluoroantimonat vorgesehen ist.

**24.** Polymerisierungsinitiator nach Anspruch 17, **dadurch gekennzeichnet**, daß als Sulfoniumverbindung p-Nitrobenzyl-4-Hydroxy-Phenylmethylsulfonium-Hexafluorophosphat vorgesehen ist.

**25.** Polymerisierungsinitiator nach Anspruch 17, **dadurch gekennzeichnet**, daß als Sulfoniumverbindung alpha-Naphtylmethyl-4-Hydroxy-Phenylmethylsulfonium-Hexafluoroantimonat vorgesehen ist.

**26.** Polymerisierungsinitiator nach Anspruch 17, **dadurch gekennzeichnet**, daß als Sulfoniumverbindung alpha-Naphtylmethyl-4-Hydroxy-Phenylmethylsulfonium-Hexafluorophosphat vorgesehen ist.

**27.** Polymerisierungsinitiator nach Anspruch 18, **dadurch gekennzeichnet**, daß als Sulfoniumverbindung alpha-Naphtylmethyl-4-(Methoxykarbonyloxy) Phenylmethylsulfonium-Hexafluoroantimonat vorgesehen ist.

**28.** Polymerisierungsinitiator nach Anspruch 17, **dadurch gekennzeichnet**, daß als Sulfoniumverbindung alpha-Naphtylmethyl-4- (Methoxykarbonyloxy) Phenylmethylsulfonium-Hexafluorophosphat vorgesehen ist.

**29.** Polymerisierungsinitiator nach Anspruch 17, **dadurch gekennzeichnet**, daß als Sulfoniumverbindung alpha-Naphtylmethyl-4- (Benzyloxykarbonyloxy) Phenylmethylsulfonium-Tetrafluoroantimonat vorgesehen ist.

**30.** Polymerisierungsinitiator nach Anspruch 17, **dadurch gekennzeichnet**, daß als Sulfoniumverbindung alpha-Naphtylmethyl-4- (Benzyloxykarbonyloxy) Phenylmethylsulfonium-Hexafluorophosphat vorgesehen ist.

**31.** Verfahren zum Polymerisieren eines kationisch polymersierbaren Stoffes, bei dem ein oder mehrere Sulfoniumsalze gemäß der in Anspruch 1 angegebenen allgemeinen Formel (I) und/oder der in Anspruch 5 angegebenen allgemeinen Formel (II) als Initiator einem oder mehreren kationisch polymerisierbaren Stoffen hinzugefügt und diese mittels Strahlung oder Wärme polymerisiert werden.

**32.** Verfahren nach Anspruch 31, **dadurch gekennzeichnet**, daß der kationisch polymerisierbare Stoff eine Verbindung mit einer Epoxy-Gruppe ist.

**33.** Verfahren nach Anspruch 31, **dadurch gekennzeichnet**, daß der kationisch polymerisierbare Stoff eine kationisch polymersierbare Vinyl-Verbindung ist.

**34.** Verfahren nach Anspruch 31, **dadurch gekennzeichnet**, daß der kationisch polymerisierbare Stoff eine Verbindung mit einer oder mehreren Spiro-Ortho-Ester-Gruppen, Bizyklo-Ortho-Ester-Gruppen oder Spiro-Ortho-Karbonat-Gruppen ist.

**35.** Verfahren nach Anspruch 31, **dadurch gekennzeichnet**, daß als Strahlung ultraviolette Strahlen vorgesehen ist.

**36.** Verfahren nach Anspruch 31, **dadurch gekennzeichnet**, daß die Polymerisation bei einer Temperatur oberhalb 20° C durchgeführt wird.

**37.** Verfahren nach Anspruch 31, **dadurch gekennzeichnet**, daß als Strahlung Sonnenlicht vorgesehen ist.

**38.** Verfahren nach Anspruch 31, **dadurch gekennzeichnet**, daß 0,01 bis 20 Gewichtsteile eines Sulfoniumsalzes gemäß der in Anspruch 1 angegebenen allgemeinen Formel (I) und/oder der in Anspruch 5 angegebenen allgemeinen Formel (II) 100 Gewichtsteilen des kationisch polymerisierbaren Stoffes hinzugefügt werden.

**Revendications**

**1.** Un composé de sulfonium représenté par la formule générale (I) suivante:

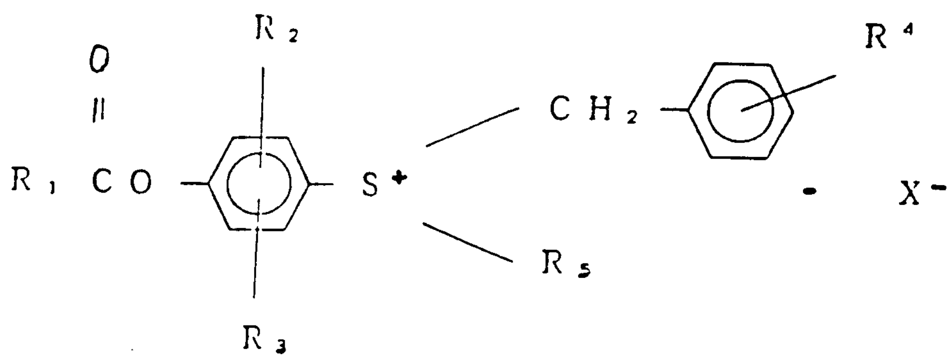

…… ( I )

caractérisé en ce que $R_1$ représente un groupe éthoxy, un groupe phényle, un groupe benzyloxy, un groupe phénoxy ou un groupe 9-fluroénylméthoxy, $R_2$ et $R_3$ représentent, indépendamment, hydrogène, halogène ou un groupe alkyle $C_1$-$C_4$, $R_4$ représente un hydrogène ou un groupe méthoxyle, $R_5$ représente un groupe alkyle $C_1$-$C_4$ et $X^-$ représente $SbF_6$, $PF_6$, $AsF_6$ ou $BF_4$.

**2.** Un composé sulfonium selon la revendication 1, caractérisé en ce que le composé sulfonium est un benzyl-4-(benzyloxycarbonyloxy)phénylméthylsulfonium hexafluoroamtinonate.

**3.** Un composé sulfonium selon la revendication 1, caractérisé en ce que le composé sulfonium est un benzyl-4-(éthoxycarbonyloxy) phénylméthylsulfonium hexafluoroamtinonate.

**4.** Un composé sulfonium selon la revendication 1, caractérisé en ce que le composé sulfonium est un benzyl-4-(phenoxycarbonyloxy) phénylméthylsulfonium.

5. Un composé sulfonium représenté par la formule générale (II) suivante:

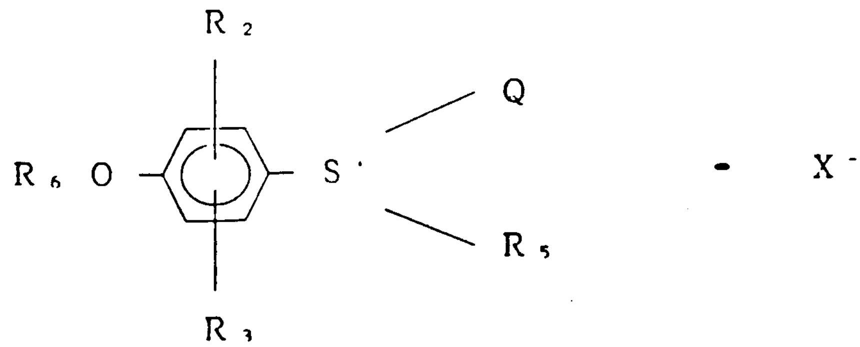

… (II)

caractérisé en ce que $R_2$ et $R_3$ représentent, indépendamment, un hydrogène, un halogène ou un groupe alkyle $C_1$-$C_4$, $R_5$ représente un groupe alkyle, $R_6$ représente un hydrogène, un groupe méthyle, groupe acéthyle, un groupe méthoxycarbonyle, un groupe éthoxycarbonyle, un groupe chloroacétyle, un groupe trichloroacétyle, un groupe benzyloxycarbonyle, un groupe benzoyle, un groupe phénoxycarbonyle ou un groupe 9-fluorénylméthoxylcarbonyle, Q représente un groupe chlorobenzyle, un groupe méthylbenzyle, un groupe nitrobenzyle, un groupe dinitrobenzyle, un groupe $\alpha$-naphthylméthyle ou un groupe $\beta$-naphthylméthyle et X représente $SbF_6$, $PF_6$, $AsF_6$ ou $BF_4$, et en ce que quand $R_6$ est un hydrogène, un groupe méthyle, un groupe acétyle ou un groupe méthoxycarbonyle, Q ne peut pas comporter un groupe chlorobenzyle ou un groupe méthylbenzyle.

6. Un composé sulfonium selon la revendication 5, caractérisé en ce que le composé sulfonium est un p-méthylbenzyl-4- (benzyloxycarbonyloxy) phénylméthylsulfonium hexafluoroantimoniate.

7. Un composé sulfonium selon la revendication 5, caractérisé en ce que le composé sulfonium est un p-méthylbenzyl-4- (benzyloxycarbonyloxy) phénylméthylsulfonium hexafluorophosphate.

8. Un composé sulfonium selon la revendication 5, caractérisé en ce que le composé sulfonium est un p-nitrobenzyl-4- hydroxyphenylmethylsulfonium hexafluoroantimoniate.

9. Un composé sulfonium selon la revendication 5, caractérisé en ce que le composé sulfonium est un p-nitrobenzyl-4- hydroxyphénylméthylsulfonium hexafluorophosphate.

10. Un composé sulfonium selon la revendication 5, caractérisé en ce que le composé sulfonium est un $\alpha$-naphthylmethyl-4- hydroxyphenylméthylsulfonium hexafluoroantimoniate.

11. Un composé sulfonium selon la revendication 5, caractérisé en ce que le composé sulfonium est un $\alpha$-naphthylmethyl-4- hydroxyphénylméthylsulfonium hexafluorophosphate.

12. Un composé sulfonium selon la revendication 5, caractérisé en ce que le composé sulfonium est un $\alpha$-naphthylméthyl-4-(métoxycarbonyloxy)-phénylméthylsulfonium hexafluoroantimate.

13. Un composé sulfonium selon la revendication 5, caractérisé en ce que le composé sulfonium est un $\alpha$-naphthylmethyl-4-(métoxycarbonyloxy)-phénylméthylsulfonium hexafluorophosphate.

14. Un composé sulfonium selon la revendication 5, caractérisé en ce que le composé sulfonium est un $\alpha$-naphthylméthyl-4-(benzyloxycarbonyloxy)-phénylméthylsulfonium hexafluoroantimoniate.

15. Un composé sulfonium selon la revendication 5, caractérisé en ce que le composé sulfonium est un $\alpha$-naphthylméthyl-4-(benzyloxycarbonyloxy)-phénylméthylsulfonium hexafluorophosphate.

16. Un composé sulfonium selon la revendication 1 caractérisé en ce que le composé sulfonium est un benzyl-4-(benzyloxycarbonyloxy) phenylmethyl-sulfonium hexafluoroantimoniate.

17. Un amorceur de polymérisation pour une matière polymérisable cationiquement caractérisé en ce qu'il comprend un sel sulfonium représenté par la formule générale (I), comme définie dans la revendication 1 et/ou par la formule générale (II) comme définie dans la revendication 5.

18. Un amorceur de polymérisation comme défini dans la revendication 17, caractérisé en ce que le composé sulfonium est un benzyl-4-(benzyloxycarbonyloxy) phénylméthylsulfonium hexafluoroantimoniate.

19. Un amorceur de polymérisation comme défini dans la revendication 17, caractérisé en ce que le composé sulfonium est un benzyl-4-(éthoxycarbonyloxy)phénylméthylsulfonium hexafluoroantimoniate.

20. Un amorceur de polymérisation comme défini dans la revendication 17, caractérisé en ce que le composé sulfonium est un benzyl-4-(phénoxycarbonyloxy)phénylméthylsulfonium hexafluoroantimoniate.

21. Un amorceur de polymérisation comme défini dans la revendication 17, caractérisé en ce que le composé sulfonium est p-méthylbenzyl-4-(benzyloxycarbonyloxy)phénylméthylsulfonium hexafluoroantimoniate.

22. Un amorceur de polymérisation comme défini dans la revendication 17, caractérisé en ce que le composé sulfonium est p-méthylbenzyl-4-(benzyloxycarbonyloxy)phénylméthylsulfonium hexafluorophosphate.

23. Un amorceur de polymérisation comme défini dans la revendication 17, caractérisé en ce que le composé sulfonium est p-nitrobenzyl-4-hydroxy-phénylméthylsulfonium hexafluoroantimoniate.

24. Un amorceur de polymérisation comme défini dans la revendication 17, caractérisé en ce que le composé sulfonium est un p-nitrobenzyl-4-hydroxy-phénylméthylsulfonium hexafluorophosphate.

25. Un amorceur de polymérisation comme défini dans la revendication 17, caractérisé en ce que le composé sulfonium est un $\alpha$-naphthylmethyl-4-hydroxy-phénylméthylsulfonium hexafluoro- antimoniate.

26. Un amorceur de polymérisation comme défini dans la revendication 17, caractérisé en ce que le composé sulfonium est un $\alpha$-naphthylmethyl-4-hydroxy-phénylméthylsulfonium hexafluorophosphate.

27. Un amorceur de polymérisation comme défini dans la revendication 18, caractérisé en ce que le composé sulfonium est un $\alpha$-naphthylmethyl-4-(méthoxycarbonyloxy)phénylméthylsulfonium hexafluoroantimoniate.

28. Un amorceur de polymérisation comme défini dans la revendication 17, caractérisé en ce que le composé sulfonium est un $\alpha$-naphthylmethyl-4-(methoxycarbonyloxy)phénylméthylsulfonium hexafluoroantimoniate.

29. Un amorceur de polymérisation comme défini dans la revendication 17, caractérisé en ce que le composé sulfonium est un -naphthylmethyl-4-(benzyloxycarbonyloxy)phénylméthylsulfonium tétrafluoroantimonate.

30. Un amorceur de polymérisation comme défini dans la revendication 17, caractérisé en ce que le composé sulfonium est un -naphthylméthyl-4-(benzyloxycarbonyloxy)phénylméthylsulfonium hexafluorophosphate.

31. Un procédé de polymérisation pour une matière polymérisable cationiquement, qui comprend l'addition d'un ou plusieurs sels sulfonium représentés par la formule générale (I) comme définie dans la revendication 1 et/ou par la formule générale (II) comme définie dans la revendication 5 comme un amorceur pour une ou plusieurs matières polymérisables cationiquement et polymérisées par un moyen de radiation à faisceaux et/ou à chaleur.

**32.** Un procédé comme défini dans la revendication 31, caractérisé en ce que le matière cationiquement polymérisable est un composé ayant un groupe époxy.

**33.** Un procédé comme défini dans la revendication 31, caractérisé en ce que la matière polymérisable cationiquement est un composé vinyle polymérisable cationiquement.

**34.** Un procédé comme défini dans la revendication 31, caractérisé en ce que la matière polymérisable cationiquement est un composé possédant un ou plusieurs groupes spiro-ortho ester, bicyclo-ortho ester ou spiro-ortho carbonate.

**35.** Un procédé comme défini dans la revendication 31, caractérisé en ce que les rayonnements sont des rayons ultraviolets.

**36.** Un procédé comme défini dans la revendication 31, caractérisé en ce que la polymérisation est faite à une température de 20° C

**37.** Un procédé comme défini dans la revendication 31, caractérisé en ce que les rayonnement sont la lumière solaire.

**38.** Un procédé comme défini dans la revendication 31, caractérisé en ce que de 0.01 à 20 parties pondérales du sel sulfonium représenté par la formule générale (I) comme définie dans la revendication 1 et/ou par la formule générale (II) comme définie dans la revendication 5 est additionné à 100 parties pondérales d'une matière polymérisable cationiquement.